(19) Europäisches Patentamt
European Patent Office
Office européen des brevets

(11) **EP 4 392 231 B1**

(12) **EUROPEAN PATENT SPECIFICATION**

(45) Date of publication and mention
of the grant of the patent:
**01.07.2026 Bulletin 2026/27**

(21) Application number: **22859659.9**

(22) Date of filing: **25.08.2022**

(51) International Patent Classification (IPC):
*B29C 64/106* (2017.01)    *B29L 31/00* (2006.01)
*B33Y 10/00* (2015.01)    *B33Y 80/00* (2015.01)
*C12N 5/00* (2006.01)    *A61L 27/24* (2006.01)

(52) Cooperative Patent Classification (CPC):
**B33Y 80/00; A61L 27/24; B29C 64/106;
B33Y 10/00; B33Y 70/00; C12M 3/00**

(86) International application number:
**PCT/AU2022/050999**

(87) International publication number:
**WO 2023/023772 (02.03.2023 Gazette 2023/09)**

(54) **COLLAGEN BIOMATERIAL DERIVED FROM ABALONE**

KOLLAGENBIOMATERIAL AUS ABALON

BIOMATÉRIAU DE COLLAGÈNE DÉRIVÉ D'ORMEAU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.08.2021 AU 2021902788**

(43) Date of publication of application:
**03.07.2024 Bulletin 2024/27**

(73) Proprietor: **Bio Consultancy Pty Ltd
Chapel Hill, Queensland 4069 (AU)**

(72) Inventors:
• **MANICKAVASAGAM, Bhanumathy
Chapel Hill, Queensland 4069 (AU)**
• **TETSWORTH, Kevin Dorland
Fig Tree Pocket, Queensland 4069 (AU)**

(74) Representative: **Buzzi, Notaro & Antonielli d'Oulx
S.p.A.
Corso Vittorio Emanuele II, 6
10123 Torino (IT)**

(56) References cited:
WO-A1-02/102851        WO-A2-2019/098585
KR-A- 20160 058 538    NZ-A- 544 672
US-A1- 2004 167 318    US-A1- 2007 179 283
US-A1- 2008 167 447    US-A1- 2020 179 532

## Description

### TECHNICAL FIELD

The present invention is concerned with a manufactured article prepared from a biomaterial comprising isolated and purified type I collagen fibrils derived from abalone and a method of preparing such an article. The manufactured article can be prepared by 3D bioprinting, and therefore the present invention also envisages a novel bioink for a 3D bioprinter comprising an isolated and purified type I collagen fibril and cartridges containing such a bioink. The invention additionally relates to a method of isolating type I collagen fibrils from abalone. The manufactured article is useful in a number of biomedical applications including as an implantable device where it can function as a 3D bioprinted hard or soft tissue scaffold for tissue engineering.

### BACKGROUND

Disease, injury and trauma can lead to damage and degeneration of tissues in the human body, which necessitates treatments to facilitate their repair, replacement or regeneration. The field of regenerative medicine (a term used interchangeably with tissue engineering) is an approach which aims to regenerate the damaged tissues instead of replacing them, by implanting biological substitutes such as biomaterials that restore, sustain or improve tissue function. Regenerative medicine has the potential to provide a cure to failing or impaired tissues and relies considerably on the use of porous three-dimensional (3-D) scaffolds to provide a suitable environment for the regeneration of tissues and organs.

A biomaterial is any material that comprises the whole or part of a living structure or biomedical device and performs, augments, or replaces a natural function to improve the quality of life of the patients (Tathe et al., 2010). Biomaterials are used in medical devices, particularly in those applications for which the device either contacts or is temporarily inserted or permanently implanted in the body. The material selection requirements are determined by the specific device application. For soft tissue device applications, the materials are typically implanted into soft tissue to augment or redefine the tissue. In orthopaedic and dental applications, the materials are components of structural implants or are used to repair bony defects. As a lifesaving and life-improving option for countless patients, biomaterials have been paid more and more attention during the last decade.

Where implants and medical devices are concerned, biomaterials range from simple implants like intraocular lenses (which restore sight to millions of cataract patients every year), sutures, wound dressings, decellular matrices, bone plates, joint replacements to more complex materials like biosensors, catheters, pacemakers, blood vessels, artificial heart (that provide both mechanical and biological functions in a body), left ventricular assist devices and prosthetic arterial grafts.

The aim of regenerative medicine is to restore or replace damaged or diseased tissues with healthy, functioning tissue. The process of tissue engineering often begins with a scaffold, which is a three-dimensional support medium essential for the appropriate proliferation and differentiation of cells embedded in, or infiltrating, the scaffold. Pre-made porous soft and hard tissue scaffolds for tissue engineering (TE) are made of degradable biomaterials has become the most used and well-established scaffolding approach. Scaffolds are materials that have been engineered to cause desirable cellular interactions to contribute to the formation of new functional tissues for medical purposes. Cells are often 'seeded' into these structures capable of supporting three-dimensional tissue formation. Many types of biomaterials can be used to make porous scaffolds for tissue engineering provided that a fabrication technology compatible with the biomaterial properties is available. In general, biomaterials used for making porous scaffolds for tissue engineering can be natural polymers which are derived from mainly from animal sources (such as collagen, silk, chitosan), synthetic polymers (such as polyglycolic, polylactic acid, and poly-N-iso-propylacrylamide), metals and alloys (such as stainless steel, silver, gold, and titanium) and ceramics (such as calcium phosphates, and bioactive glasses) and composites (such as polyvinyl chloride-glass fillers).

The selection of a suitable biomaterial must be determined for every case, considering the type and amount of tissue being regenerated, stability and geometric conformation of the area, vascularization of the adjacent tissues, possible inflammatory response normally associated with foreign bodies and the capacity of this material to be biodegradable and replaced by new bone tissue. Furthermore, when considering a biomaterial for implantation or medical use, the first and most important requirement is nontoxic, non-immunogenic, chemically inert/active, and acceptable by the human body. Therefore, important criteria to consider when fabricating hard and soft tissue scaffolds for TE are biocompatibility, biodegradability, pore interconnectivity, pore size, porosity, and mechanical properties. Biocompatible in most cases means that the biomaterials must not form thrombi in the blood system, result in tumors in the surround tissues, or be immediately attacked, encapsulated, or rejected by the body (Patel et al.,2012).

Naturally occurring materials are those that are produced by the cells of living organisms. The most common and abundant naturally occurring biomaterial is collagen. In its native state collagen is a natural substrate for cellular attachment, growth and differentiation. Collagen Type I is the most abundant collagen protein present in the human body and is found within skin, ligaments, tendons and bone. Collagen biomaterial have been used for a range of tissue engineering applications, including tendon, dermal, neural, brain, adipose, dental and cartilage tissue engineering. Bone's native composition is predominantly type I collagen and accounts for 90% of bone matrix protein content.

Traditionally, most collagen is extracted from the skins of cattle (bovine) and pigs (porcine), sheep (ovine) or fish (piscine) waste. Among natural polymers, bovine hydrolysed collagen (HC), primarily that of type I, has long been used in biomedical applications. The major disadvantage of using bovine hydrolysed collagen are allergic reactions and transmission of pathogens such BSE, TSE, FMD and avian influenza. Furthermore, bovine hydrolysed collagen from porcine and bovine are unacceptable to some religions. In addition, from an orthopaedic perspective, mammalian hydrolysed collagen scaffolds are limited by their poor mechanical characteristics. These problems can be overcome through enhanced cross-linking mechanisms to improve the strength, stiffness and stability of the construct. However, the cross-linking process results in unintended changes to cell viability, adhesion or proliferation on the treated structures and often forms inferior tissues. The process of cross-linking also results in further modifications to the molecular structure of collagen. Furthermore, the collagen molecule is somewhat different across species and there is the possibility of an immune response when a vertebrate animal derived hydrolysed collagen-based product is used in humans.

While alternative sources of collagen biomaterial have been explored, the market is currently lacking a collagen biomaterial that is disease free, meets scaffold criteria for repair, and industrial translation of the extraction and manufacturing processes to meet the safety standards applicable to biomedical applications. Hence selection of a starting material, which will somehow mimic a naturally existing one, is one of the most important points and crucial elements in biomaterial development.

Extraction of collagen from vertebrate animal (such as bovine, porcine, ovine or piscine) source requires hydrolysis by the action of proteolytic enzymes (alcalase, papain, pepsin, and others). The resulting product is commonly called hydrolysed collagen and is composed of small peptides.

The triple-helix structure of native collagen changes to a random coil form due to the dissociation of the hydrogen bonds when bovine collagen undergoes hydrolysis. This treatment breaks the bonds in the polypeptide chain to obtain a large number of peptides. The molecular weight of bovine collagen peptides obtained from hydrolysis is very low (3-6 KDa) compared to that of its precursor native collagen (285-300 KDa). Enzymatic hydrolysis affects not only the size of the peptides but also physicochemical and biological properties (Zhang et al., 2017 and Li et al.,2013). Viscosity is one of the physicochemical properties of collagen; the native form shows higher values due to stronger electrostatic repulsion among the molecular chains even at low concentrations of collagen solution. However, its hydrolysed form shows very low viscosity no matter the concentration because of the low molecular weight of the small chain segments (Sun et al.,2018). Electrostatic properties of proteins such as the isoelectric point (pI) are important parameters which are related to the proportion of acid amino residues and base amino residues in protein. Collagen is an amphoteric macromolecule that possesses a pI value between 7 and 8. On the hydrolysis process, the pI value is shifted to lower values between 3.68 and 5.7. This change will depend on the amino acid sequences and distribution of amino acid residues according to the type or time of hydrolysis. Hydrolysed collagen is not able to form films by itself. It is necessary to combine it with other biopolymers (Fauzi et al., 2016 and Ramadass et al., 2014).

Hydrolysed collagen-based biomaterials are usually generated through cross-linking reaction due to the hydrolysed collagen instability and poor mechanical properties. There are three types of polymerisation techniques that reinforce the hydrolysed collagen structure: physical, chemical, and enzymatic cross-linking. Chemical cross-linking techniques include treating the isolated hydrolysed collagen with glutaraldehyde, genipin (a chemical compound obtained from the iridoid glucoside, geniposide), 1-Ethyl-3-(3-dimethylaminopropyl) carbodiimide (EDC) and N-hydroxysuccinimide (NHS), dialdehyde starch (DAS) or chitosan. The strength, resorption rate, and biocompatibility of hydrolysed collagen cross-linked biomaterials are profoundly influenced by the method and extent of cross-linking.

In US 2004/0167318 A1 the isolation of collagen type I from Abalone is disclosed, wherein a weak acid and salting-out are used in the process, followed by dialysis to remove the salt.

Accordingly, there remains a need for a biomaterial with chemical, physical, biological, and mechanical properties at least equivalent to commercial bovine hydrolysed collagen cross-linked biomaterial.

## SUMMARY OF THE INVENTION

The present inventors have identified a biomaterial that can produce a 3D collagen scaffold with biological, chemical, and mechanical properties superior to manufactured articles such as scaffolds produced from commercial cross-linked bovine hydrolysed collagen.

In a first aspect the present invention provides a method of preparing a manufactured article, comprising the steps of:

a) providing a plurality of isolated and purified type I collagen fibrils derived from abalone;
b) forming the isolated and purified type I collagen fibrils into a manufactured article which contains ions or salts, preferably sodium chloride, within an intrafibrillar compartment.

In a second aspect the present invention provides a bioink for a 3D bioprinter comprising isolated and purified type I collagen fibrils derived from abalone and a solvent, wherein the bioink has a predetermined concentration of abalone collagen fibrils and a predetermined viscosity that is low enough that the bioink can be bioprinted at a temperature below the denaturation temperature of abalone collagen and wherein a printed article contains ions or salts within an intrafibrillar compartment.

In a third aspect the present invention provides a manufactured article such as a scaffold for tissue engineering, comprising a plurality of isolated and purified type I collagen fibrils derived from abalone in which there is association of collagen molecules from adjacent collagen fibrils and which contains ions or salts, preferably sodium chloride, within an intrafibrillar compartment.

In a fourth aspect the present invention provides a method of isolating type I collagen fibrils from abalone, comprising the steps of:

a) preparing a collagen-containing portion of abalone for extraction;

b) contacting the collagen-containing portion with a weak acid solution to provide an acidic type I collagen fibril solution;

c) separating insoluble material from the acidic type I collagen fibril solution;

d) adjusting the pH of the acidic type I collagen fibril solution to a pH just below the isoelectric point of abalone collagen, preferably to about 4.0;

e) precipitating native type I collagen fibrils by addition of a salt, preferably sodiumchloride; wherein the precipitated native type I collagen fibrils are not dialysed against water.

## BRIEF DESCRIPTION OF THE DRAWINGS

Embodiments of the invention will now be described with reference to the accompanying drawings, in which:

Figure 1 shows the denaturation temperature of isolated and purified type I collagen fibrils derived from abalone collagen (referred to herein as MCol-B) - Sample A (lyophilized MCol-B) and Sample B (non- lyophilised MCol-B);

Figure 2 shows the results of an *in vitro* degradation study of MCol-B films, showing (A) Water uptake (%). (B) Water content (%) and (C) Mass loss (%). Error bars represent Mean $\pm$ SE;

Figure 3 shows (A) CD spectrum of abalone collagen (in the Far UV region (195-260 nm), (B) CD spectrum of abalone collagen in the near UV region (260-320 nm).

Figure 4 shows the results of Fourier Transform - Infrared spectroscopy depicting the IR spectrum of the samples between 4000 and 450 cm$^{-1}$ (A) untreated MCol-B film. (B) MCol-B samples incubated in PBS at 37°C over a period of 21 days;

Figure 5 shows the results of tensile testing of abalone collagen films. Stress- strain % curve of collagen film samples in replicates;

Figure 6 is graph showing attachment of cells (A: NIH-3T3 and B: BHK) to calf skin collagen (CSC) and abalone collagen (MCol-B) on nontreated polystyrene in the absence of fetal calf serum (serum);

Figure 7 shows attachment of cells (A:hASC and B:BHK) to abalone collagen (MCol-B) and calf skin collagen (CSC) on treated polystyrene tissue culture plate;

Figure 8 presents photomicrographs of human stem cells (hASC) attached to abalone collagen (MCol-B) and calf-skin type I collagen (CSC) in presence and absence of 10% fetal calf serum (serum). Note more cells attached to abalone collagen (MCol-B) under both conditions, while cells seem to be more spread on bovine collagen;

Figure 9 shows attachment of cells (A. BHK, B. NIH3T3, and C. hASC) to bovine collagen (CSC) and abalone collagen (MCol-B) on polystyrene tissue culture plastic wells;

Figure 10 is a photomicrograph which shows human adipose stem cells growing at 48 hours within a 3D lattice (gel) formed from abalone collagen in DMEM;

Figure 11 shows rat 8 mm CSDs treated with MCol-B Gels. Top, an X-Ray image of untreated rat skull defect, showing no bone regeneration during the 6-week study. Bottom row: X-Rays of CSDs of 5 rats implanted with 15 mg/ml Abalone collagen gels for 6 weeks. Healing ranged from 75% in rat A to 25% or less in the other 4 (B-E). Asterisks indicate regions of new bone formation;

Figure 12 shows the histology of regenerated bone in rat CSD treated for 6 weeks with 15 mg/ml MCol-B gel. Arrow heads denote interface of host bone defect and newly formed tissue. Arrows indicate region of new bone formation and areas of fibers of collagen, presumably MCol-B gel, that has persisted for the 6-week experimental period; and

Figure 13 is a photograph of 3D printed abalone collagen (MCol-B) constructs: (A) a line print prepared from dialysed abalone collagen (B) a square with infills prepared from non-dialysed abalone collagen and (C) a meniscus prepared from dialysed abalone collagen.

## DETAILED DESCRIPTION

The present invention relates to a manufactured article comprising a plurality of isolated and purified type I collagen fibrils derived from abalone. The collagen has a high molecular weight. In such a manufactured article the collagen fibrils self-associate and connections form between adjacent collagen molecules.

In contrast, extraction of collagen from bovine sources requires hydrolysis by the action of proteolytic enzymes (alcalase, papain, pepsin, and others). The resulting product is commonly called hydrolysed collagen and is composed of small peptides with low molecular weight 3-6 KDa. The triple-helix structure of native collagen changes to a random coil form due to the dissociation of the hydrogen bonds when bovine collagen undergoes hydrolysis. This treatment breaks the bonds in the polypeptide chain to obtain a large number of peptides. The molecular weight of collagen peptides obtained from hydrolysis is very low (3-6 KDa) compared to that of its precursor native collagen (285-300 KDa). Enzymatic hydrolysis affects not only the size of the peptides but also physicochemical and biological properties ((Zhang et al., 2017 and Li et al., 2013). Viscosity is one of the physicochemical properties of collagen; the native form shows higher values due to stronger electrostatic repulsion among the molecular chains even at low concentrations of collagen solution. However, its hydrolysed form shows very low viscosity no matter the concentration because of the low molecular weight of the small chain segments (Sun et al.,2018). Electrostatic properties of proteins such as the isoelectric point (pI) are important parameters which are related to the proportion of acid amino residues and base amino residues in protein. Collagen is an amphoteric macromolecule that possesses a pI value between 7 and 8. Following the hydrolysis process, the pI value is shifted to lower values between 3.68 and 5.7. This change will depend on the amino acid sequences and distribution of amino acid residues according to the type or time of hydrolysis. Hydrolysed collagen is not able to form films by itself and is necessary to combine it with other biopolymers (Fauzi et al., 2016).

Therefore, unlike bovine collagen, which is degraded during isolation and must have a chemical cross-linking agent introduced to restore its physical properties, native type I abalone collagen can be formed into manufactured articles in the absence of a cross-linking agent.

Manufactured articles comprising native type I abalone collagen are useful in medical applications including orthopaedic medicine, augmentation techniques, wound care, cosmetics, sponges, injectables and drug delivery. In particular, uses of the biomaterial include 3D bioprinting of hard tissue (bone) and soft tissue (skin), and manufacture of implantable item such as a patch for rotator cuff repair. The invention relates further to a bioink comprising a plurality of isolated and purified type I collagen fibrils derived from abalone and to scaffolds prepared by 3D bioprinting.

The process of isolating abalone collagen fibrils involves treatment with dilute acids, alkali and salt to provide pure intact native type I collagen fibril as a biomaterial that is free from DNA, residual lipids and any accompanying contaminants, and which is not cross-linked. This provides a safe, effective and natural native non cross-linked abalone collagen fibril biomaterial that mimics both the chemical and mechanical properties of native tissues, which is key to the success of any scaffold for regenerative medicine applications. Thus, the present invention deals, not with reconstituted collagen, but with isolated and purified type I abalone collagen fibril from the starting material as a novel biomaterial for biomedical applications.

Since the abalone lives 10 meters below the pristine clean Australian ocean the abalone is disease free and is a suitable collagen biomaterial for medical use. Foot muscle of abalone usually contain large amounts of fibril-forming collagen. Therefore, these biological features of abalone foot muscles are likely to be at least partly related to the molecular properties of their abundant fibril-forming collagens. The collagen in abalone foot muscle is highly cross-linked and shows a collagen content-dependent toughness which changes in various muscle parts.

Abalone foot or pedal consists of two distinct regions, the columellar muscle region and the tarsic region. The columellar region consists primarily of muscle-fiber bundles that are oriented parallel to the long axis of the muscle in at least two directions. The tarsic region consists of bundles of muscle fibers that branch and change directions as they extend from their origins to their insertions. They form a complex three-dimensional network of interconnecting contractile fibers. Both the columellar muscle and tarsic regions have thick connective tissue sheaths surrounding the muscle fibers. These characteristics of abalone foot muscle seem to be correlated with their adaptation for locomotion from a variety of muscle wave propagations. There are two types of fibril-forming collagen in abalone foot muscle. Early collagen fibrils can be unipolar (with all molecules pointing in the same direction) or bipolar (in which the orientation of collagen molecules reverses at a single location along the fibril). The occurrence of such early fibrils has major implications for tissue morphogenesis and repair. Collagen fibril occurrence is not only in vertebrates but also in invertebrates. Invertebrate bipolar fibrils are centro-symmetrical (with the molecular switch region at the midpoint of the fibril), whereas those in vertebrate are shape-asymmetrical, thus the mechanism of assembly of bipolar fibrils is different in vertebrates and invertebrates.

Type I collagen is a fibrillar type collagen. Collagen fibers appear to be composed of bundles of threadlike subunits called collagen fibrils. Type I collagen is a heterotrimer molecule. In most cases it is composed of two $\alpha$1 chains

and one $\alpha$2 chain, although an $\alpha$1 homotrimer exists as a minor form. Each chain consists of more than 1000 amino acids, and the length of a collagen type I molecule is ~300nm and the width about 1-5nm. It has three domains: an N-terminal non-triple helical domain (N-telopeptide), a central triple helical domain, and a C-terminal non-triple helical domain (C-telopeptide), of which the central domain is by far the largest, comprising approximately 95% of the total molecule. The triple helical domain is only possible due to the presence of glycine (G)-X-Y repeats, where X often is a proline and Y is a hydroxyproline. Glycine at every third position is essential for the correct formation of the structure. Lysine and hydroxylysine residues in some positions, including the C terminus, are cross-linked between the individual helices, leading to the formation of extensive covalent intra- and intermolecular cross-links.

Thus, fibrils comprise collagen molecules assembled into the fibril through intermolecular cross-links (mainly covalent bonds). In nature, these long, thin fibrils cross-link to one another in the spaces around cells. The cross-links result in the formation of very strong mature type I collagen fibre, and these cross-links are broken down to allow isolation of collagen fibrils. Collagen fibrils formed mainly from type I collagen (all fibrous tissues except cartilage) and fibrils formed largely from type II collagen (cartilage) have slightly different structures. The nature and extent of cross-linking depend on the physiological function and age of the tissue. With age, the density of cross-linkages increases, rendering connective tissue rigid and brittle. Since the extracted abalone collagen is in native state it retains the collagen fibril structures whereas, as described above, bovine collagen is hydrolysed during extraction and cannot form collagen fibrils.

The isolation process generally comprises treating a collagen-containing portion of abalone with a weak acid solution to solubilise the type I collagen fibrils. According to the present invention the process comprises the steps of:

a) preparing a collagen-containing portion of abalone for extraction;

b) contacting the collagen-containing portion with a weak acid solution;

c) adjusting the pH of the solution to a pH below the isoelectric point of abalone collagen;

d) precipitating native type I collagen fibrils by sodium chloride addition.

In an embodiment the weak acid solution is an acetic acid solution, typically a 0. 1M to 1M solution, preferably a 0.5M solution. A weak acid is one with a dissociation constant between 1.0 x 10~5 and 1.0 x 10~2 in aqueous solution and so is predominantly un-ionised. Suitable weak acids may be readily identified by the person skilled in the art but include lactic, butyric, formic, propionic and citric acids.

In an embodiment the pH of the weak acid solution is adjusted to about 3.5. This may be done through addition of a strong acid, such as 0.1N HCL.

Typically, the pH of the type I collagen fibril solution is about 3.5 and at this pH the collagen is in solution. Solid matter can be removed from the type I collagen fibril solution in any suitable manner, as will be well understood by the person skilled in the art. In an embodiment solid matter is separated by centrifugation.

Typically, an alkali is added to adjust the pH in step (d). The pl of abalone collagen is about 4.5. Accordingly, the pH is adjusted to just below 4.5, typically to about 4.

The present process may be contrasted to using isoelectric point precipitation for abalone collagen especially in large scale manufacture. The greatest disadvantage of isoelectric point precipitation is the irreversible denaturation caused by the acids. Therefore, isoelectric point precipitation is better suited to precipitate contaminant proteins rather than the target protein (abalone collagen).

Hence, the pH of the abalone collagen supernatant is advantageously adjusted to about pH 4 (below but close to the isoelectric point of abalone collagen). While not wishing to be bound by theory, it is believed that the collagen has a net positive charge and offers cationic sited for the salts to bind and promote precipitation of collagen. Thus, the abalone collagen molecules assemble into fibrils by an entropy driven process caused by the loss of solvent molecules which leads to an energetically minimized area/volume ratio. The salt precipitation also removes the non-collagenous proteins such as lipids and DNA and other materials and concentrates the collagen fibrils.

Advantageously, the pH adjustment takes place after the collagen-containing portion has been in contact with the weak acid solution for two to twelve hours.

Antigenicity of collagen is related to the presence of non-collagenous proteins (DNA and lipids). The extraction method used for abalone collagen fibril eliminates lipids and DNA.

Advantageously, the collagen-containing portion is spun down in a centrifuge and native collagen is precipitated from the supernatant. Additional collagen may be extracted from the pellet, if desired. In a typical collection process from the supernatant, sufficient sodium chloride, typically in solid form, is added to bring the supernatant to 0.3M sodium chloride.

The native collagen may be purified in a further purification step. Suitable means for purification of proteins are well understood by the person skilled in the art. In an embodiment, purification is by buffer exchange against deionised water using an ultrafiltration membrane, typically a 100kD ultrafiltration membrane.

In order to collect native collagen fibrils, the temperature should preferably be maintained at or below 25°C, and more preferably at or below 4°C.

Advantageously, the collagen-containing portion is muscle tissue, which has preferably had pigment removed therefrom. This may be achieved by soaking the intact muscle tissue in a weak acid solution. The weak acid solution is typically an acetic acid solution, preferably a 0.2M solution.

Preferably the abalone is a commercial species such as the black-lip abalone, Haliotis ruber, the brown-lip abalone Haliotis conicopora and the green-lip abalone, Haliotis laevigata, or Roe's abalone, Haliotis roei.

In an embodiment collagen is extracted from juvenile green lip abalone, which is advantageous due to the ease of extracting the collagen fibrils.

Native abalone collagen fibrils are useful in a variety of applications. In particular, native abalone collagen fibrils are useful in the preparation of manufactured articles. Manufactured articles can be prepared in a variety of ways, depending on the nature of the article and the use to which it will be put. By way of example, native abalone collagen fibrils may be cast as films, moulded into articles or 3D printed to form shaped articles.

In an embodiment the manufactured article is formed by:

a) dissolving the isolated and purified type I collagen fibril in a solvent to produce a collagen bioink with a predetermined concentration of type I collagen fibrils and a predetermined viscosity;

b) introducing the collagen bioink to a 3D bioprinter;

c) printing the manufactured article in the 3D bioprinter while maintaining a relatively constant temperature to maintain the predetermined viscosity.

In an embodiment the temperature that is maintained is greater than 0°C and less than or equal to 15°C. Desirably, the temperature is maintained at 4°C. A temperature below 15°C ensures that polymerisation of collagen fibril to a gel does not take place prematurely.

In an embodiment the viscosity of the collagen bioink is from 10 mPa-S to 60mPa-S. A viscosity in this range ensures that the collagen bioink is printable and will produce a product with adequate mechanical strength. Advantageously the viscosity of the collagen solution is 50mPa-S.

In an embodiment the concentration of the collagen bioink is from 3mg/ml to 20 mg/ml. the concentration of the collagen bioink and the temperature at which printing takes place may be controlled in tandem to ensure that this solution has an appropriate viscosity for a particular solvent. Advantageously the concentration of the collagen solution is 8mg/ml.

In an embodiment the collagen bioink is an aqueous solution typically at pH 3.5. The solution may comprise an acid selected from the group consisting of acetic, citric, propionic, lactic, butyric and formic acid.

In a further aspect the invention relates to a bioink for a 3D bioprinter comprising an isolated and purified type I collagen fibril derived from abalone dissolved in a solvent such that a collagen bioink with a predetermined viscosity is produced, and the predetermined viscosity is low enough that the bioink can be bioprinted at a temperature below the denaturation temperature of abalone collagen.

An effective bioink should possess excellent mechanical properties and should not breakdown post-printing. Current commercial collagen bioink formulations are not printable and exhibit poor mechanical properties. Hence the collagen in commercial formulations is cross-linked to form scaffolds or films. As discussed above, cross-linking of collagen causes a number of negative biological effects.

Before commencing bioprinting, bioink needs to be developed. Bioink developments are one of the most challenging issues in the 3D bio-printing process. Generally, the bioink must fulfil the biological, physical and mechanical requirements of the printing process. Firstly, from a biological aspect, the bioink should be biocompatible whilst allowing cell adhesion and proliferation. Physically, the bioink requires a viscosity low enough to dispense from the print head. Finally, the paramount mechanical requirement is to provide sufficient strength and stiffness to maintain structural integrity of the bioink after printing. While not wishing to be bound by theory, it is believed that small molecules such as sodium and chloride ions can easily penetrate into the intrafibrillar compartment of collagen in accordance with the present invention and, consequently, create a stiffer collagen structure. Salts affect the thermal stability of collagen in a typical ion-specific way; they alter the structure of the solvent, which, consequently, modifies the solvent-macromolecule interaction involved in the stabilization of the natural conformation.

The manufactured article of the invention comprising type I collagen fibril contains ions or salts within the intrafibrillar compartment. In an embodiment, sodium chloride is contained within the intrafibrillar compartment. In an embodiment sodium chloride used in the isolation process is retained and encapsulated within the intrafibrillar compartment.

In an embodiment the viscosity of the collagen bioink is from 10mPa-S to 60mPa-S. A viscosity in this range ensures that the collagen bioink is printable and will produce a product with adequate mechanical strength. Advantageously the viscosity of the collagen bioink is 50mPa-S.

A bioink is typically provided in a cartridge adapted to fit into a 3D bioprinter in order to deliver bioink for printing. Such a cartridge may be refillable, or the bioink may be delivered directly into the 3D bioprinter.

In an embodiment the bioink is deposited onto a surface, such as by depositing into a glass petri dish, in a layer-by-layer process to build 3D constructs analogous to tissues or organs. There are two basic methods of bioprinting: drop based and extrusion. In drop-based bioprinting, sometimes referred to as inkjet bioprinting owing to its origins in modified inkjet bioprinters, a mechanical print head deposits the bioink in droplets that can then coalesce and gel to form polymeric structures. Extrusion bioprinting utilizes a mechanical extruder to continuously deposit the bioink as the extruder or stage is moved.

In an embodiment, the bioink is adapted for extrusion bioprinting.

In an embodiment cells or cell aggregates/spheroids may be incorporated in the bioink.

In an embodiment the manufactured article is formed by:

a) dissolving the isolated and purified type I collagen fibril to produce a collagen solution at room temperature or below;
b) shaping the collagen solution; and
c) inducing polymerisation of collagen molecules to form a hydrated gel;

wherein steps (a) and (b) are carried out at a temperature below that at which the collagen molecules polymerise and polymerisation is induced by way of an increase in temperature. The manufactured article contains ions or salts, preferably sodium chloride, within an intrafibrillar compartment.

In an embodiment the collagen solution is an aqueous solution. The pH of the collagen solution produced in step (a) is acidic, typically with a pH as low as 3.5.

When shaping articles such as by moulding it is desirable for the pH of the collagen solution to be as close as possible to physiological pH. Accordingly, in in an embodiment, the pH of the collagen solution is adjusted to physiological pH of 7.4. Typically, the pH of the collagen solution is adjusted by the addition of a base such as sodium hydroxide or sodium bicarbonate, or mixtures of the two. Alternatively, the pH of the collagen solution may be adjusted by equilibrating the solution by dialysis against a cell culture medium.

It is desirable when forming shaped articles for steps (a) and (b) to carried out room temperature or below. At these temperatures polymerisation of collagen molecules to form a hydrated gel will not be induced to any significant extent. Desirably, steps (a) and (b) are carried out at 4°C.

In an embodiment polymerisation is induced by exposing the collagen solution to a temperature greater than 4°C and less than or equal to 37°C.

In an embodiment polymerisation is induced by exposing the collagen solution to a temperature greater or equal to 15°C and less than or equal to 25°C.

In an embodiment polymerisation is induced by exposing the collagen solution to room temperature.

In an embodiment polymerisation is induced by exposing the collagen solution to a temperature of 37°C. The shaped article can be any article. Typically, the collagen solution is cast as a sheet, or the collagen solution is moulded. When moulding or casting a collagen solution it is desirable that it have a higher concentration of collagen than when bioprinting. The collagen solution has a concentration of greater than 35mg/ml.

In an embodiment the manufactured article comprises a further biomaterial. In order to prepare such a biomaterial, in an embodiment, a solution of the further biomaterial is mixed with the collagen solution. The biomaterial can be any material that may be formed into a shaped article along with abalone collagen fibrils. Desirably, the additional biomaterial enhances a physical property of the shaped article. The additional material can be selected from the group consisting of alginate, carrageenan, propylene glycol, xanthan gum, gum Arabic and hydroxyapatite. In an embodiment the further biomaterial is alginate.

Individual collagen polypeptide chains have a large number of repeating amino acid sequences, and form an extended, left-handed triple helix, which is longer and less compact than the $\alpha$-helices often seen in proteins. Three of these helixes then form a molecule of tropocollagen, the basic building block of collagen, by coiling around a central axis in a right-handed, triple-helical arrangement. Tropocollagen molecules associate in a staggered fashion form a collagen fibril. Collagen fibrils are strengthened and stabilized mainly by covalent cross-links, existing both within and between individual tropocollagen molecules. It will be appreciated that the use of purified and isolated abalone collagen fibrils in manufacturing an article without addition of cross-linking agents results in a novel structure. Accordingly, the invention relates to a manufactured article comprising a plurality of isolated and purified type I collagen fibrils derived from abalone in which there is association of collagen molecules from adjacent collagen when fibrils are brought together without addition of a cross-linking agent as when forming biomaterials from hydrolysed collagen.

The natural cross-linking pathway of lysyl oxidase is responsible for mechanical resilience of collagen-containing tissues and their proteolytic resistance (Smith-Mungo et al., 1998). The aggregated forms of the collagen monomers are stabilised to provide mechanical strength by a series of intermolecular cross-links. These links are formed by oxidative deamination of the $\varepsilon$-amino group of the single lysine in the amino- and carboxy-telopeptides by lysyl oxidase. The aldehyde thus formed reacts with an $\varepsilon$-amino group of a lysine at a specific point in the triple helix due to the quarter-staggered end- overlap alignment of the molecules in the fibers. The chemistry of these cross-links is dependent on both the nature and the age of the collagenous tissue (Bailey et al., 1989 and Knott et al., 1998). Differences in the cross-links are due to the degree of hydroxylation of both the telopeptide and the specific lysine in the triple helix. Thus, the amounts of inter- mediate cross-links present in immature tissue, dehydro-hydroxylysine or norleucine ($\Delta$-HLNL) and hydroxylysino-keto-norleucine (HLKNL) may vary considerably between tissues, e.g., rat tail tendon and skin contain $\Delta$-HLNL whilst cartilage and bone contain predominantly HLKNL.

These divalent cross-links are only intermediates and are subsequently converted into stable trivalent cross-links that accumulate in the tissue as collagen turnover decreases during maturation (Knott et al., 1998). The Schiff base aldimine $\Delta$-HLNL is stabilised by reaction with histidine to form the trivalent cross-link, histidino-hydroxlysinonorleucine (HHL). The keto-imine HLKNL, on the other hand, reacts with a second hydroxylysyl aldehyde to form the pyridine derivatives, hydroxylysyl-pyridinoline (Hyl-Pyr) and lysyl-pyridinoline (Lys-Pyr). The proportion of these three known mature cross-links, again varies with the age and with the type of tissue. For example, HHL is the major, known, mature cross-link of human and bovine skin (Yamauchi et al.,1987), whilst the pyridinolines are the major, mature cross-links of bone and cartilage (Eyre et al, 1980); tendon, however, contains a mixture of both mature cross-links. A high value for the immature cross-links indicates predominantly new collagen synthesis, as in growing tissue, fibrosis, wound healing and bone fracture repair. Conversely a predominance of the mature cross-links indicates low collagen turnover.

While not wishing to be bound by theory, it is believed that cross-links form between collagen molecules that form part of adjacent fibrils when a plurality of isolated and purified type I collagen fibrils derived from abalone are formed into a manufactured article. This may be contrasted to hydrolysed collagen, where a cross-linking agent is added.

As used herein, the term "polymerisation", unless the context requires otherwise, refers to the formation of a network of native type I abalone collagen fibrils.

In addition to interacting with biomacromolecules, collagen acts as a ligand for cell receptors. Biological activity of collagen is mediated through integrin receptors, which interact with the high affinity ligand, GFOGER, on the helical collagen fiber. The $\beta1$ integrin subfamily is responsible for facilitating binding to collagen. Specifically, $\alpha1\beta1$, $\alpha2\beta1$, $\alpha_{10}\beta_1$, and $\alpha_{11}\beta_1$ receptors bind to the GFOGER sequence (Gly-Phe-hydroxyproline-Gly-Glu-Arg) in nondenatured collagen type 1 and binding is highly dependent on the presence of the glutamic acid within the ligand sequence.

Gelatin contains the linear RGD (Arg-Gly-Asp) cell adhesive motif. For example, when collagen is denatured, such as in the form of gelatin, or in areas of damage including wounded or fibrotic tissue, collagen is partially denatured and can support $\alpha_v\beta_3$ and $\alpha_5\beta_3$ binding via normally inaccessible RGD sequences.

While not wishing to be bound by theory, the biomaterial of the present invention is not cross-linked by a chemical reaction with a cross-linking agent and so it enhances cellular activities including attachment and proliferation through interactions between the GFOGER domains of collagen and the integrin receptors in the cell membrane. With the increased availability, GFOGER sequences in abalone collagen provide additional binding sites for cells compared with cross-linked bovine collagen films.

In an embodiment a manufactured article is a 3D bioprinted hard and soft scaffold for tissue engineering. From an orthopaedic perspective, mammalian collagen scaffolds are limited by their poor mechanical characteristics. These problems are overcome through enhanced cross-linking mechanisms, but studies have indicated that cross-linking collagen results in limited cell-material interactions and often forms inferior tissues.

In an embodiment a manufactured article such as a scaffold is used in soft tissue regeneration. Current commercial challenges using mammalian collagen include tailoring scaffold degradation and scaling up of scaffold production to treat large scale defects in a potentially high number of patients. A patch prepared by the process of the present invention has the potential to provide reinforcement that leads to lower re-tear rates and better functional outcomes. Such a patch has beneficial effects for tissue healing in many clinical applications. Collagen scaffolds are produced to mimic the tendon or ligament extracellular micro- environment, to stimulate cell proliferation and tissue in-growth. For a reinforcement patch to be efficacious in assisting in the tendon repair process, it needs to address the scaffold criteria requirements. A patch prepared by the process of the present invention is free of contaminants (lipids and DNA) that cause antigenic/immunogenic reaction and will create an environment conducive to healing. The patch maintains tissue integrity and provides the basis for enhanced mechanical properties. In addition, abalone in Australia feed on fresh seaweeds and therefore have a very low or no lipid content.

## EXAMPLES

### *Example 1 Isolation and purification of abalone collagen fibrils*

Collagen is extracted from green lip and black lip abalone sourced from Australia using a modification of the process described WO2002/102831. In embodiment collagen is extracted from juvenile green lip abalone. It is advantages due to the ease of extracting the collagen fibrils.

Abalone tissue from juvenile green lip abalone was soaked in 0.2M acetic acid overnight with slight agitation and then washed under running cold tap water to remove pigmentation from the outer areas of the epipodium, the hard part of the foot (pedal sole) and the upper part of the adductor (columellar) muscle. 500kg of cleaned minced abalone tissue was used for extraction.

In order to extract collagen fibrils, 0.5 M acetic acid solution was added to the tissue and pH adjusted to 3.5. The suspension was stirred for 2 hours then homogenized. The pH of the abalone collagen solution was then adjusted to 4.0 in order to be below, but close to, its isoelectric point. The pI of abalone collagen is 4.5. The mixture was stirred for 4 hours at 4 °C to extract collagen fibrils and the mixture was centrifuged to remove tissue particulates. In order to precipitate the native collagen fibrils, the supernatant was brought to 0.3M sodium chloride by gradually adding solid sodium chloride to the supernatant with constant stirring. Visible white collagen fibrils precipitated within 2 minutes. The remaining liquid was stirred overnight to further extract the native collagen fibrils. The solution had a high viscosity indicating the presence of collagen. The native collagen fibrils were collected by centrifugation at 5,000 rpm at 4° C for 30 minutes. The native collagen fibrils were mixed into in a minimum quantity of de-ionised water. The collagen suspension was buffer exchanged against 6 volumes of de-ionised water using a 100kD NMCO ultrafiltration membrane.

The purified native collagen fibrils were transferred into freeze drying bottles and frozen in liquid nitrogen. The samples were freeze dried for approximately 16 hours. A yield of 438 kg of freeze-dried abalone collagen was obtained. The collagen isolated in this process is referred to herein after as "MCol-B". The isolated fibrils retain an intact molecular structure: with preserved telopeptides and NH2- side groups of asparagine and glutamine amino acids. The product easily undergoes self-assembly at optimal conditions with the appearance of native banded fibrils.

Alternatively, the sodium chloride precipitated abalone collagen fibrils were not dialysed against water and were collected.

### Example 2 Preparation of MCol-B Films

This Example demonstrates achieving a smooth non-crosslinked MCol-B film structure and improving the drying kinetics of collagen films under mild conditions of temperature. Various MCol-B solution between 5mg/ml to 10mg/ml, 20mg/ml to 40mg/ml at pH 3.54 were prepared by dissolving abalone collagen in MilliQ water and the pH adjusted to 3.54 and films were cast from each solution.

The lyophilised MCol-B was dissolved in MilliQ water having a concentration of 35 mg/ml and the pH adjusted to pH 3.54. The solution was stirred on a magnetic stirrer, until the solution was completely dissolved. A solution of MCol-B was prepared as a film using a casting method. The solution was degassed for 1 hour to remove air-bubbles. The degassed collagen solution was poured into a mould preferably the mould has the property of being non-stick so that the dried collagen film will not adhere to the surface of the mould. We used a mould made of Styron (Polystyrene) of different sizes and volumes of 10ml, 20ml and 30ml were used. The MCol-B solution in the Styron mould was allowed to dry at room temperature under a laminar hood. The drying process was continued until the constant weight of the film could be obtained. The dried films formed were easy to peel off and transparent. There were no visible bubbles on the surface of the films and resulted in elastic films. The collagen films once dry were stored in a desiccator under vacuum.

The collagen solution having a concentration of 35 mg/ml (pH 3.54) provided the best condition to cast the films. It was found that collagen solution of concentrations less than 35 mg/ml yielded films which were brittle and prone to tears when immersed in Phosphate Buffered Saline (PBS) at 37 °C. It was difficult to peel the 10ml and 20ml collagen film and they were wrinkly. The 30 ml collagen solution cast in large Styron mold provided the best smooth films with no wrinkles or brittleness and were easily removed without any tear.

The MCol-B fiber bundles aligned in the plane of the film, giving rise to a stiffer structure. These conditions allow theses collagen molecules from gels to be structured and to form intermolecular bonds without any cross-linking agent. Hence without chemical crosslinking, a wide range of surface topographies, mechanical properties and cellular activities can be attained.

### Example 3 MCol-B -Alginate Composite Films

The lyophilised MCol-B was dissolved in MilliQ water having a concentration of 35 mg/ml and the pH adjusted to pH 3.54 and a 1% alginate solution was prepared in MilliQ water. 10ml of the 1% alginate solution was mixed with 10 ml of the 35mg/ml collagen solution and stirred on a magnetic stirrer for 4 hours at room temperature. The films were cast and dried as described for MCol-B films.

The 30 ml MCol-B-Alginate solution cast in large Styron molds provided the best films with no wrinkles or brittleness. The films were easily removed without any tear.

### Example 4 Differential scanning calorimetry (DSC)

### Preparation of collagen solutions

Sample 1: 40mg of lyophilized MCol-B was dissolved in 2 ml of 0.1M acetic acid at pH 3.5. The solution was stirred on a magnetic stirrer, until the solution was completely dissolved.

Sample 2: non-lyophilised stock MCol-B solution was diluted to obtain 40gm in 2ml MilliQ water. The concentration was measured by Bio-Rad Protein Assay (based on the method of Bradford).

### Reference Samples

Reference Sample 1: 3ml of 0.1M (pH 3.5) was used as a reference for lyophilised MCol-B

Reference Sample 2: 3ml of MilliQ water was used as a reference for non-lyophilised MCol-B

### Analysis

DSC was carried out by using DSC 200PC. The abalone collagen solution was degassed at 25°C for half an hour and equilibrated at $10^0$C for 2 hours before the heating process. The heat and temperature scales were calibrated according to the manufacturer's instructions.

### Results

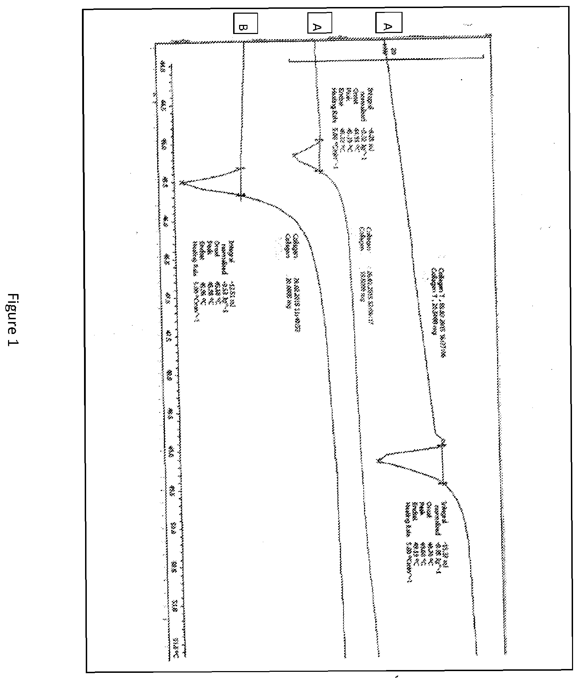

Sample 1 had a peak at 45.98°C and Sample 2 had a peak at $49^0$ C (Figure 1). The freeze drying might have shifted the melting temperature of the abalone collagen to a higher value. The apparent melting temperature was defined at the maximum of the melting peak after baseline subtraction.

The denaturation temperature range found for most vertebrate marine species range from 26°C to 29°C (Cho et al., 2014) although calf skin collagen is 39°C and human lung collagen monomers denature at 37°C (Leikina et al., 2002) within a couple of days. Their unfolding rate decreases exponentially at lower temperature, but complete unfolding is observed even below 36°C. The content of hydroxyproline is particularly relevant due to its relation to the thermal stability of collagen. It is known that hydroxyproline forms hydrogen bonds between collagen polypeptides, stabilizing the triple helix and thus a higher content of hydroxyproline would characterize a collagen exhibiting higher denaturation temperature (Rosenblo et al., 1973). The main difference between marine vertebrate and mammalian collagen are that there is a lower content of imino acids (proline and hydroxyproline) in marine vertebrate collagen, which also influences the lower thermal stability shown by fish collagens. The hydroxyproline content for avian is 98, calf skin 73, fish skin 83 but for abalone collagen is 130. Thus, abalone collagen has a high hydroxyproline content and also exhibits a high thermal denaturation temperature compared to other species.

The denaturing temperature is the point where the collagen triple helix structure is deformed to a random spiral structure. Denaturation drastically alters the physicochemical, biological, and mechanical properties of collagen. Collagen is susceptible to heat which can induce the triple helix to collapse, resulting in the thermal denaturation of collagen into gelatin. The high denaturation temperature of abalone collagen is advantageous for clinical use.

### Example 5 In-vitro degradation of collagen films

The collagen films were subjected to *in-vitro* degradation by immersing them in PBS at 37°C. Small samples of the film were cut, approximately 10 mg in weight and immersed in 30 ml of PBS containing 0.2% sodium azide, to prevent microbial contamination. The initial pH of the PBS was recorded to be 7.24 and remained unchanged over the course of the study. The initial mass ($m_i$) of the triplicate samples was recorded using a Mettler Toledo GR-202 balance before immersing them in fresh PBS. The water uptake, water content and mass loss of the samples was monitored over a 21-day period and measurements were made every three days. The wet films were dipped in MilliQ water to remove any residual salts present on the films and dabbed with Kimwipes to eliminate excess liquid from the surface before proceeding to measure the wet mass of the films ($m_w$). The dry mass of the samples was measured after allowing the samples to dry at room temperature ($m_D$). The wet mass and the dry mass of the samples were measured at definite time points. The water content was calculated using the equation (1).

$$\text{Water content (\%)} = \frac{\underline{m_W} - \underline{m_D}}{m_W} \times 100\% \quad (1)$$

The mass loss was calculated using equation (2)

$$\text{Mass loss (\%)} = \frac{mi \underline{- m_D}}{m_i} \times 100\% \quad (2)$$

The water uptake was calculated using equation (3)

$$\text{Water uptake (\%)} = \frac{\underline{m_W} - \underline{m_i}}{m_i} \times 100\% \quad (3)$$

The *in- vitro* degradation study was repeated again with new sample films in triplicates (10 mgs each approximately in weight), however, this time the water uptake, mass loss and water content were monitored only over a 7-day period to see if there were any major changes in the water uptake and water content when left immersed in PBS for one week.

### Results

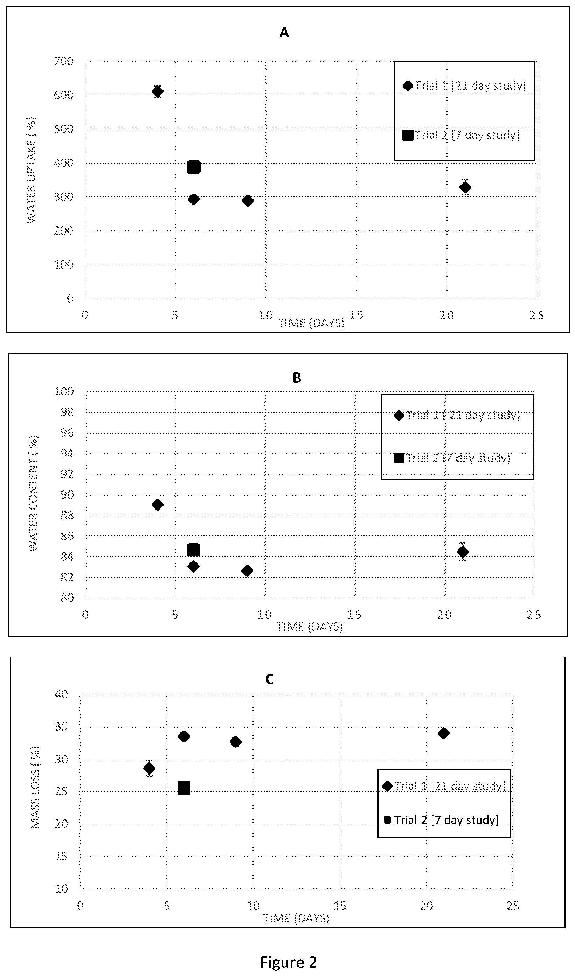

Figure 2 shows the in-vitro degradation of MCol-B films. The water uptake, mass loss and water content were estimated at each time point (Day 4, 6, 9 and 21). The swelling ability of the films and the ability to bind and absorb fluid was assessed, in addition to evaluating the degradation of the films *in- vitro* based on the mass loss. The water uptake or the swelling ability of the films seemed to reach the maximum in the first four days of ~611%, however they were found to decrease over the course of the next two weeks and reached a constant water uptake of ~329 % by the end of 21 days. A similar trend was observed with the water content, where the films showed a maximum of 89% water content on day 4 before decreasing to a constant water content of ~84% at the end of the study.

It was observed that the water uptake and water content were independent of the incubation time with PBS. However, the mass loss showed a time dependent behaviour, indicating that degradation increased with increasing period of exposure or incubation in PBS. An initial mass loss of ~28% was observed before reaching a mass loss of ~34%.

Due to the nature of changes in the water uptake and water content, a second *in-vitro* degradation study was undertaken where new films were incubated in PBS for 7 days and the water content, water uptake and mass loss was measured. The second study revealed that the water content and water uptake reached a constant of ~84% and 400% respectively on day 6. The mass loss of the second study was ~25%, validating the previous assertion that mass loss was time dependent. The water content and water binding capacity (uptake) of the films appear to be increasing before reaching a plateau and remained almost constant for the rest of the study. The ability of the films to bind fluids and retain the fluid indicate effective absorption of PBS which is a physiologically relevant buffer and mimics conditions in-vivo (Rubin et al., 1973).

### Example 6 Circular Dichroism (CD) measurements

CD measurements were carried out using the JASCO-J-715 spectropolarimeter. The CD spectra was recorded in the 190-260 nm (far UV) region and in the 260-320 nm (near UV) region to study the secondary and tertiary structures of the protein, respectively. MCol-B samples having a final concentration of 0.125 mg/ml for the far UV region and 0.5 mg/ml for the near UV region was used. Both the samples were analysed at 25°C and 0.1 M acetic acid pH 3.54 was used as the blank. Bandwidth of 0.5 nm with a scan speed of 50 nm/min and response of 2 seconds was used with an accumulation of 5 scans in a 1 mm cell.

### Results

CD is a powerful technique used to study the secondary structure of proteins based on the differential absorption of circularly polarized light. It is also widely used to study the helical content of collagen, since it contributes to the structural integrity of tissues, scaffolds and films used in tissue engineering (Norma et al., 2006). In this study, MCol-B was characterized using CD, to confirm the helical content and the expected spectrum for a collagen molecule. CD can indicate that extracted collagen is in the native-triple helical structure or denatured form-coil structure.

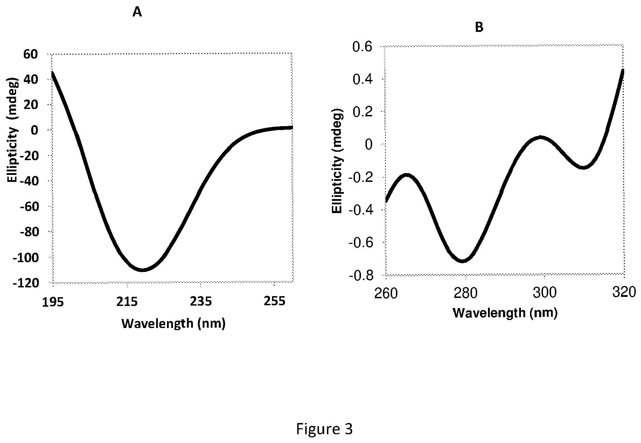

The Far UV (190-260 nm) region of the spectrum obtained from the MCol-B sample revealed a negative band at 222nm and a positive band at 195nm indicating an $\alpha$-helical protein (Figure 3 (A)). However, the spectrum failed to show the characteristic positive n-$\pi$* transition at 222nm and the negative $\pi$-$\pi$* amide transition at 200 nm, which is associated with the spectrum of a collagen molecule. This difference was evident when the results of the non-cross linked MCol-B sample was compared to the CD spectrum of cross-linked collagen and succinylated collagen, both obtained from bovine Achilles tendon (Gopal et al., 2015). The Far UV CD spectra was analysed using the K2D3 software and the MCol-B $\alpha$ helical content was found to be 95.5% and the $\beta$ strand content was found to be 0.01%. Thus MCol-B has a native triple helix structure.

The near UV region of the spectrum (260-310 nm) of the collagen sample showed a negative band at 280 nm which is characteristic of tyrosine present in the environment (Figure 3 (B)). The near UV region is characterised by the presence of aromatic amino acids like tyrosine, tryptophan and phenylalanine. The near UV spectrum provide changes to the aromatic amino acids when there are changes in the environment

## *Example 7 Spectroscopic analysis by Fourier Transform Infrared Spectroscopy*

Fourier transform infrared spectroscopy was carried out using the Perkin-Elmer Spectrum Two-FT IR spectroscopy instrument at 25 °C. The dried abalone collagen films were then placed in between the sample holder and the spectrum was recorded between 450 and 4000 $cm^{-1}$. The spectra were processed and analysed using the Spectrum 10™ software. FTIR was performed for untreated films and each of the triplicate films after drying to constant weight at the end of every time point (Day 4, 6, 9 and 21).

### *Results*

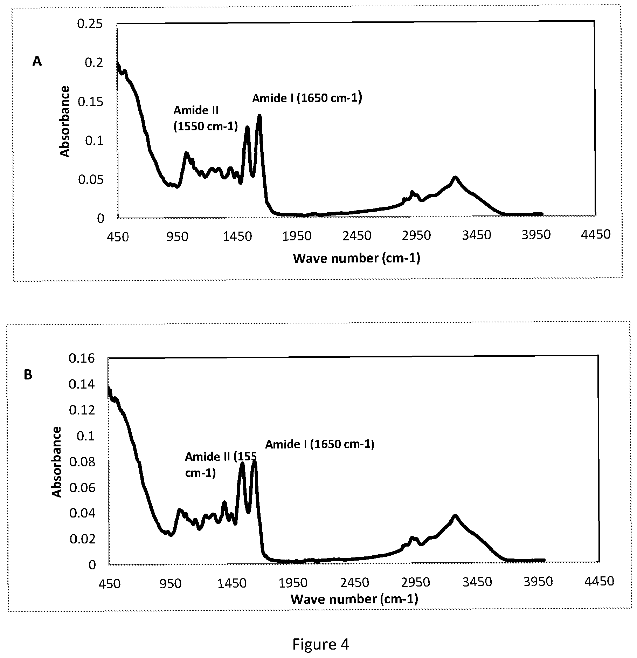

FT-IR spectra (Figure 4) revealed that, both untreated and treated collagen samples show the characteristic vibration modes of Amide A (3330 $cm^{-1}$) Amide B (1550 $cm^{-1}$), Amide I (1650 $cm^{-1}$), II (1550 $cm^{-1}$), and III (1250 $cm^{-1}$). The amide I vibration mode is caused due to the C=O stretching vibrations, the amide II and III are due to the CN stretching and NH bending vibrations (ref). Based on peak assignments in the literature for the spectral features of collagen, the peak observed near 1650 $cm^{-1}$ indicates denatured collagen (Habermehl et al., 2005). The spectral features for the untreated and the treated collagen samples appear almost unchanged, However, in order to check if there was any rearrangement or reordering of the structure, the ratios of $A_{1650}/A_{1026}$ and $A_{1550}/A_{1026}$ was estimated for the treated and untreated samples and compared. The $A_{1650}/A_{1026}$ and $A_{1550}/A_{1026}$ ratios were found to increase from 1.63 to 2.00 and from 1.38 to 1.75 respectively (Table 1), when the collagen film samples were subject to treatment in PBS. The increase in ratios of $A_{1650}/A_{1026}$ and $A_{1550}/A_{1026}$ suggests the presence of an ordered secondary protein structure, after treatment. This also indicates that incubation of the film samples in PBS over the course of the *in-vitro* degradation study has not affected the structure of the collagen molecule.

**Table 1.** $A_{1650}/A_{1026}$ and $A_{1550}/A_{1026}$ ratios measured from the FT-IR curves, fitted to the Amide I and Amide II IR features of collagen film samples before and after treatment with PBS.

| Amide band | Absorbance of bands (Untreated sample) | Absorbance of bands (Treated sample) |
|---|---|---|
| $A_{1650}$ | 0.13 | 0.08 |
| $A_{1550}$ | 0.11 | 0.07 |
| $A_{1026}$ | 0.08 | 0.04 |
| $A_{1650}/A_{1026}$ | 1.63 | 2.00 |
| $A_{1550}/A_{1026}$ | 1.38 | 1.75 |

Distribution of secondary structure varies among marine and mammalian source. Secondary structure differs among fish species as well. The diversity of secondary structure among different collagen sources is believed to be due to the amino acid composition of the collagen which plays a major role in the secondary structure distribution.

## *Example 8 Tensile Testing of MCol-B Films*

The mechanical properties of the MCol-B films were assessed using the Instron 5848 micro tester machine. Samples were cut into strips in triplicates having dimensions about 45 mm x 10mm. The thickness of the sample films was measured using a micrometer at three different points along the length and their average thickness was 80-100 $\mu$m. Grip

pressure of about 400 KPa was applied to the ends of the films. A load of 25 N was applied and the tensile tests were carried out at a displacement rate of 15 mm/min. The stress- strain curve was generated, and the Young's modulus, ultimate tensile strength and elongation were calculated by using the Instron software. The stress- strain curve was exported to Microsoft excel and replotted for each of the replicates.

### Results

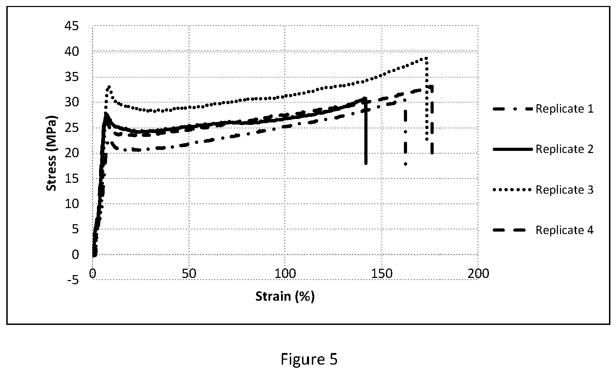

The Stress- strain curve was plotted for the four replicates. The Young's modulus was determined to be 748 $\pm$ 133 MPa, Ultimate tensile strength (Maximum tensile stress of 33 $\pm$ 4 MPa and tensile strain of 163 % (Figure 5). The Ultimate tensile strength and the tensile strain obtained for the non-cross linked MCol-B films were compared to those of cross-linked bovine type I collagen films (Gopal et al., 2016). It was found that the Ultimate tensile strength obtained for MCol-B films (33 $\pm$ 4 MPa) was more than that obtained for bovine collagen films (14.29$\pm$ 1.8 MPa) indicating the ability of the MCol-B films to withstand high stress before breaking. The MCol-B films were also able to be stretched to a higher degree before breaking, with a tensile strain of 162.9% when compared to the cross-linked bovine collagen films having a tensile strain of 16.7%. (Table 2). The high Young's modulus of 748$\pm$ 133 MPa obtained for the MCol-B films further confirms the rigidity of the material, indicating that a relatively large force would be needed to deform it. The comparison of tensile properties of MCol-B films against the cross-linked bovine collagen films revealed that the MCol-B films displayed higher mechanical properties for TE.

**Table 2.** Table comparing the Young's Modulus, Ultimate tensile stress, and tensile strength of cross-linked bovine Achilles type I collagen (Gopal et al., 2015) with abalone type I collagen.

| Source | Young's Modulus (MPa) | Ultimate tensile strength (MPa) Mean$\pm$ SD | Tensile strain (%) | Reference |
|---|---|---|---|---|
| Bovine Achilles type I collagen (cross-linked) | - | 14.29$\pm$ 1.8 | 16.7 | (Gopal Shankar et al., 2015) |
| Abalone type I collagen | 748$\pm$ 133 | 33 $\pm$ 4 | 162.9 | - |

### Example 9 Cell Attachment to Two-Dimensional (2D) MCol-B

### Methods:

Materials:

1) sample A: Lyophilised MCol-B was reconstituted (1mg/ml) in 0.1M acetic sample B: Stock solution prepared from lyophilized MCol-B, 1 mg/ml in 0.1M acetic acid.

2) Sigma: Type 1 calf skin collagen (CSC), 1mg/ml in 0.1M acetic acid.

3) Costar #3596 96 well tissue culture plates

4) Human adipose stem cells (hASCs).

5) Baby hamster kidney cells (BHK-21(C13).

6) NIH-3T3 mouse embryonic fibroblasts.

7) BrdU labeling kit, (Roche Molecular Biochemicals, Indianapolis, IN).

Coating of plates:

Sample A: MCol-B (lyophilised) 1mg/ml in 0.1M acetic acid

Sample B: Stock solution prepared from lyophilized MCol-B, 1 mg/ml in 0.1M acetic acid

Samples A and B and calf skin collagen (CSC) were diluted in ice cold Milli-Q water to 0.1,1, 5, 10 and 20$\mu$g/cm$^2$ (for later experiments 40, 60, 80 and 100 $\mu$g/cm$^2$ coating concentrations were also employed) and added to plates in triplicate wells, 100$\mu$l/well, and allowed to coat plates overnight at 37°C or at room temperature. In the morning (18 hrs), wells were gently rinsed with isotonic, Hank's buffered saline

(HBBS), and non-coated areas of plates were blocked with 2% bovine serum albumin (BSA) in PBS for 30 minutes prior to cell attachment studies.

Attachment assay: Cells were added to plates at 2x10$^4$ cells/well and allowed to attach for 1 hour. Assays were stopped by washing plates 2 times with Hanks and fixing with 4% paraformaldahyde for 15 min. For quantification, in initial experiments plates were stained with 0.1% Crystal Violet in ethanol for 15 minutes. Plates were washed 4 times with phosphate buffered saline. Then plates were read in a microtiter plate reader at 405nm to quantify the attached cells in each well. When it became clear that crystal violet binding to MCol-B at concentrations over 10 $\mu$g/cm$^2$ created high background, we switched to incorporation of the thymidine analog BrdU, which was incorporated in cells the night before attachment assays and detected by ELISA assay by manufacturer's instructions.

**Experiment 1:**

Attachment of cells to calf skin collagen (CSC) and abalone collagen (MCol-B) on nontreated polystyrene in the absence of serum.

Attachment assays to test coating concentration of MCol-B compared to calf-skin collagen (CSC) were first done in Costar non tissue culture treated (Petri-type) 96-well polystyrene plates. The concentrations tested were 0.1, 5,10, 20, 40 g/cm$^2$. Dilutions were made in sterile MilliQ water and plates were dried overnight in the tissue culture hood. Plates were washed 2 times with Hanks buffer before use. No blocking was done because these were not tissue culture-treated plates. Each of three cell types, BHK-21, and NIH-3T3, were added at 2x10$^4$/well with or without 10 % fetal calf serum.

**Experiment 2:**

Attachment of hASC and BHK cell lines on tissue culture treated polystyrene with blocking of non-coated sites by 2% BSA. Quantification of attachment by ELISA for BrdU incorporated the previous night.

**Results**

***Experiment 1: Comparing the coating of polystyrene tissue culture surfaces and ability to promote cell attachment of MCol-B with calf-skin collagen (CSC) in cell attachment assays with and without 10% serum.***

Attachment of 3 cell lines (human adipose stem cells, BHK and NIH-3T3): attachment in serum free media, in the presence of 10% bovine serum, on tissue culture and Petri type polystyrene surfaces was investigated. Methods for coating the plates with acid-solubilized MCol-B were developed along with an improved assay for quantifying cell attachment to MCol-B using BrdU labeling of cells. Attachment of all 3 different types of cells to collagen is significantly greater to MCol-B than calf skin collagen with and without the presence of 10% fetal calf serum. This suggests great utility for MCol-B in cell culture, especially in serum-free conditions, and the potential for MCol-B as a material for scaffold construction for tissue engineering applications, avoiding the necessity for serum or other vertebrate proteins.

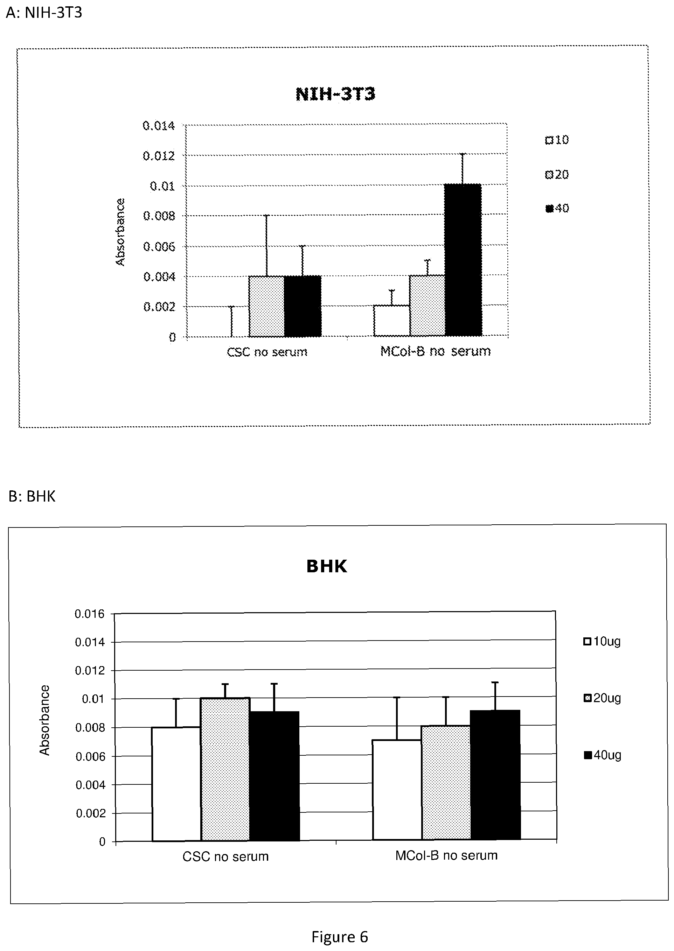

BHK cells (Figure 6) on CSC coated plates showed attachment not significantly different in the 10-40g/cm2 range (lower concentrations did not give significant signals) with little attachment in the no serum condition. The MCol-B attachment was still increasing at the 40g concentration though not much as the CSC. The no serum condition on the MCol-B had better attachment, which was also still increasing at 40$\mu$g using NIH-3T3 cells (comparable results with BHK) in Figure 6.

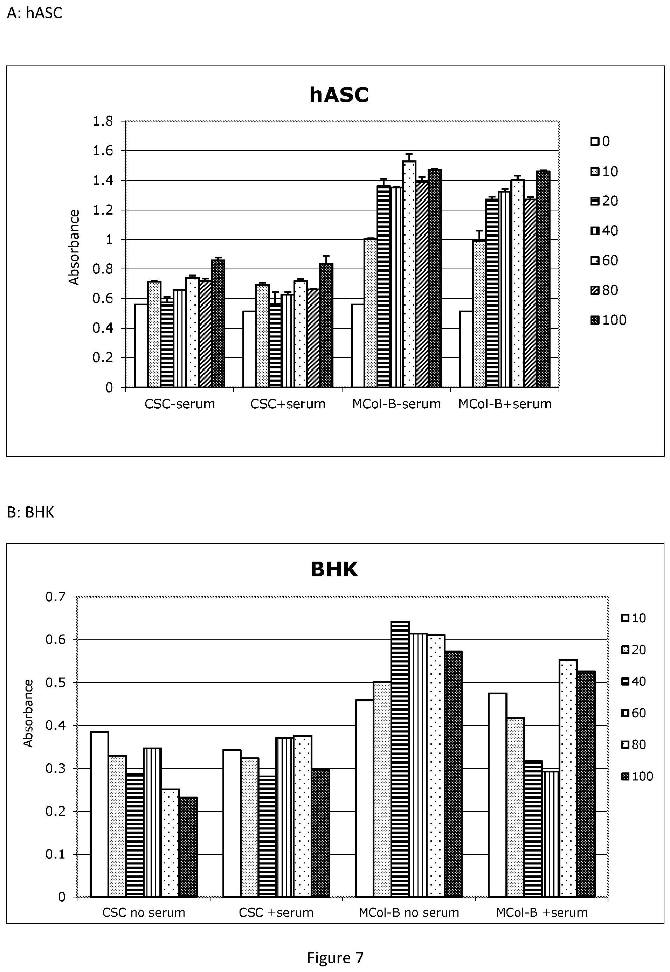

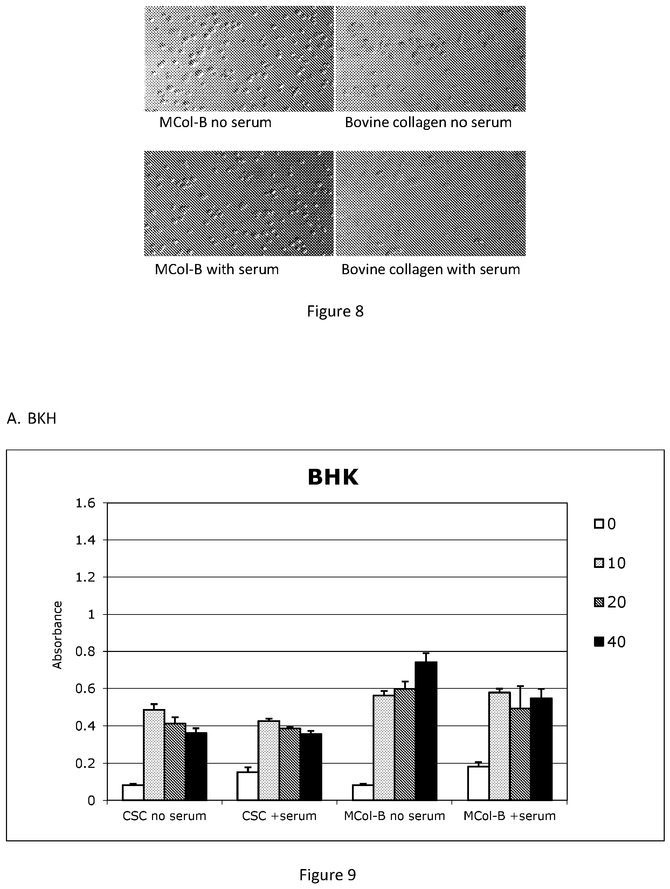

The assay was changed to labeling the cells with BrdU and detecting incorporation in the attached cells. Cells attach to MCol-B (Figure 7) as well or better without serum as with serum except at the lowest concentration tested (10$\mu$g). Cells attach to the MCol-B without serum better than to CSC without serum. As can be seen in Figures 6-8, MCol-B supported better attachment of all cell lines than calf-skin collagen with and without serum. This was most dramatic for epithelial (BHK) and human stem cells (hASC) but was also true for fibroblasts (NIH-3T3). As seen in Figure 8, more cells attached to MCol-B under both conditions, while cells seem to be more spread on bovine collagen.

### Experiment 2: Comparing the ability of MCol-B, with calf skin collagen in supporting substrate attachment of a variety of cell lines.

Attachment assays to test coating concentration of MCol-B compared to calf skin collagen (CSC) was done in Costar tissue culture treated 96-well polystyrene plates. The coating concentrations tested were 0,10, 20 and 40 mg/cm$^2$. Each of three cell types, BHK-21, NIH-3T3 or hASC, were added at $2x10^4$/well with or without 10 % fetal calf serum.

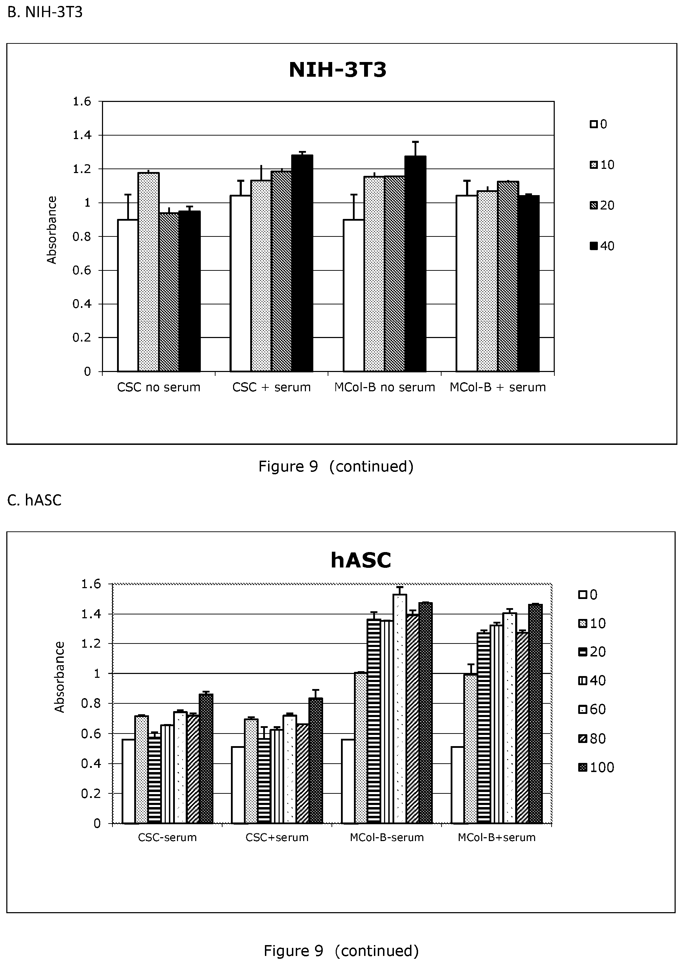

As can be seen in Figure 9, MCol-B supported better attachment of all cell lines than calf skin collagen with or without serum. This was most dramatic for epithelial (BHK) and human stem cells (hASC) but was also true for fibroblasts (NIH-3T3). Attachment of 3 different types of cells to collagen is significantly greater to MCol-B than CSC with and without the presence of 10% fetal calf serum. The adhesion mediated by MCol-B is comparable to that seen by glycoproteins such as laminin, fibronectin or vitronectin-much greater than that seen with vertebrate collagens. This finding suggests great utility for MCol-B in cell culture, especially in serum-free conditions, as well as the potential for MCol-B as a material for scaffold construction for tissue engineering applications, avoiding the necessity for serum or other vertebrate proteins while retaining the superior structural properties of collagen.

### Example 10 Formation of Heat-Induced Gels in Physiological Buffers

Experiments were conducted to determine the best methods to form heat induced, isotonic gels with abalone collagen capable of serving as three-dimensional substrates for cells in-vitro.

A series of experiments was first done with lyophilised abalone collagen dissolved in 0.1M acetic acid at 2.5 and 5.0mg/ml (stock solution prepared from lyophilized abalone collagen, and later at 15mg/ml and 7.5mg/ml. To grow cells in abalone collagen gels requires abalone collagen in a physiological buffer, therefore steps were taken to equilibrate the solution by dialysis for 2 hours at 4 degrees C against incomplete DMEM (iDMEM, without bicarbonate), which is adjusted with acid (1M HCl) to pH 2.9.

Solutions were kept at 4°C or at room temperature while mixing, then shifted to 37° to induce polymerisation of collagen monomers to form a hydrated gel. It was necessary to neutralize the solution before forming a gel. NaOH and bicarbonate (NaCO$_3$) were added to bring the solution to physiological pH (7.4).

### Results

Initially gels were observed as drops on tissue culture plastic dishes. NaOH was added to abalone collagen and formed a uniform gel that maintained its shape and got more solid overnight in at 37°C. Mixing half of an NaOH solution with all of a NaCO$_3$ solution (5μl and 11μl, respectively, per 100 μl total), then mixing with abalone collagen and finally stirring in the remaining 5 μl of NaOH formed a usable gel.

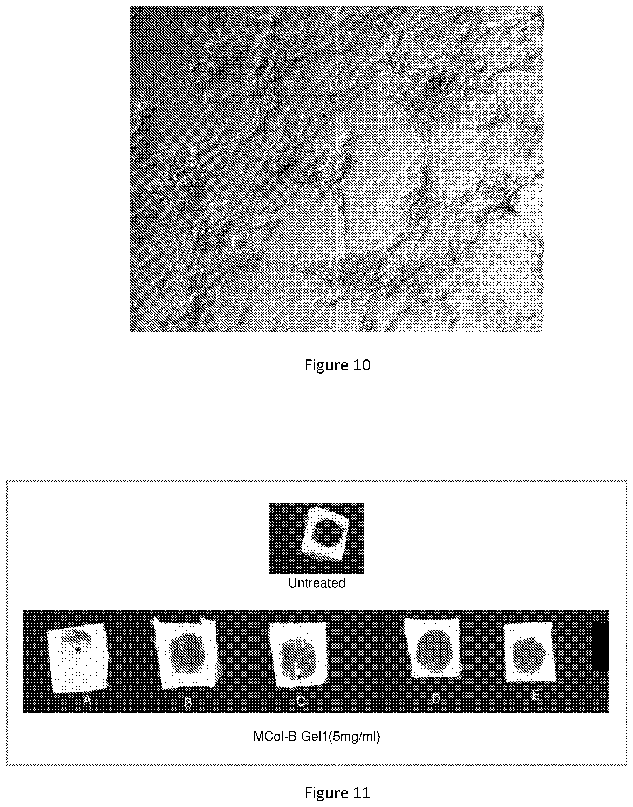

Better and stronger gels were formed from higher concentrations of abalone collagen. Also, smaller volume gels were produced, thinner in the vertical dimension, in a 96 well plate. In this method, only enough abalone collagen gel to provide a uniform coating of the surface is used, with the excess gelling solution pipetted away prior to gelation. Visually, the percentage volume of the gel occupied by cells increased with this method. Figure 10 illustrates human adipose stem cells growing within a thin abalone collagen gel after 48 hours in-vitro.

### Example 11 Cell Attachment Studies -MCol-B 3D Gel

Abalone collagen (15mg/ml) was dissolved in 0.5 M acetic acid and dialyzed against unbuffered DMEM and pH was adjusted with acid (HCl) to pH 2.9. Rat collagen type I was prepared as described for abalone collagen. Costar #3596 96 well tissue culture plates was used, and cells used were rat dura mater stem cells (RDCs) and human adipose stem cells (hASCs).

Abalone collagen and rat type I collagen were diluted in Milli-Q water to 0.1,1, 5, 10 and 20μg/ml and added to plates in triplicate wells, 100μl/well, and allowed to coat plates overnight at 37°C or at room temperature. In the morning (18 hrs), wells were gently rinsed with isotonic, phosphate buffered saline (PBS), and non-coated areas of plates were blocked with 2% bovine serum albumin (BSA) in PBS for 30 minutes prior to cell attachment studies.

Cells were added to plates at $2x10^4$ cells/well and allowed to attach for 1 hour. Assays were stopped by washing plates 2 times with Hanks and fixing with 4% paraformaldahyde 15 min. Plates were stained with 0.1% Crystal Violet in ethanol for 15 minutes. Plates were washed 4 times with phosphate buffered saline. Then plates were read in a microtiter plate reader at 405nm to quantify the attached cells in each well.

2D cell cultures poorly imitate the conditions in-vivo. Three-dimensional (3D) cell culture improves the structure of cells and physiological equivalence of in-vitro experiments. It refers to the culture of living cells inside micro assembled devices with a 3D structure mimicking tissue and organ specific microarchitecture. In 3D cell culturing, growth of cells in their 3D physical shape allows better cell-to-cell contact and intercellular signaling networks. The 3D environment also facilitates development processes allowing cells to differentiate into more complex structure.

***Example 12 In-Vivo Studies - Using MCol-B to promote bone tissue engineering in rat skull defects***

The formation and use of abalone collagen gels to mediate repair of rat 8 mm skull critical sized defects (CSDs) were studied.

*MCol-B gel preparation:*

MCol-B (15 mg/ml) in ice-cold 0.5-M acetic acid was dialyzed overnight against unbuffered Dulbecco's modified Eagle's medium and sterilized by addition of 0.1% chloroform during dialysis. To form gels, MCol-B (15 mg/ml) in 0.5 M acetic acid was mixed with 5 x concentrated Dulbecco's modified Eagle's medium in purified water.

*Operative procedure*

Animals were anesthetized by intraperitoneal injection of 17 mg/kg sodium pentobarbital and intramuscular injection of 10 mg ketamine. The hair over the calvaria was shaved and cleaned with depilatory. Lidocaine (0.5 ml of 1%) was injected intradermally in the midline on top of the head. The rats were placed in a cephalostat and the skin was incised in the midline. Using an operating microscope, the subcutaneous fascia was divided, the periosteum was incised in the midline, and periosteal flaps were reflected laterally.

An 8-mm circular CSD was templated and excavated centrally over the parietal bones using a drill with saline irrigation. Extreme care was taken not to damage the dura mater. After copious irrigation with normal saline, heat-induced gels of abalone collagen (15mg/ml) gels were placed in the defects (except in controls), and the defects were completely covered by closure of the periosteum using 10-0 nylon suture. The skin was closed using 4-0 nylon suture.

*Animal Model, Experimental Design, and Data Analysis*

Six rats, 6- to 7-month-old, retired breeder, Sprague-Dawley rats, weighing 410 to 576 g each, were randomly assigned. One animal in Group 1 (control) received no treatment for the calvarial defects. Group 2 (five animals) was implanted with the abalone collagen gel.

*Computerized tomography & Histology*

Abalone collagen was evaluated by multiple computerized tomography (CT) scans bi-weekly from 2 to 6 weeks, bone repair was evaluated using CT at 6 weeks.

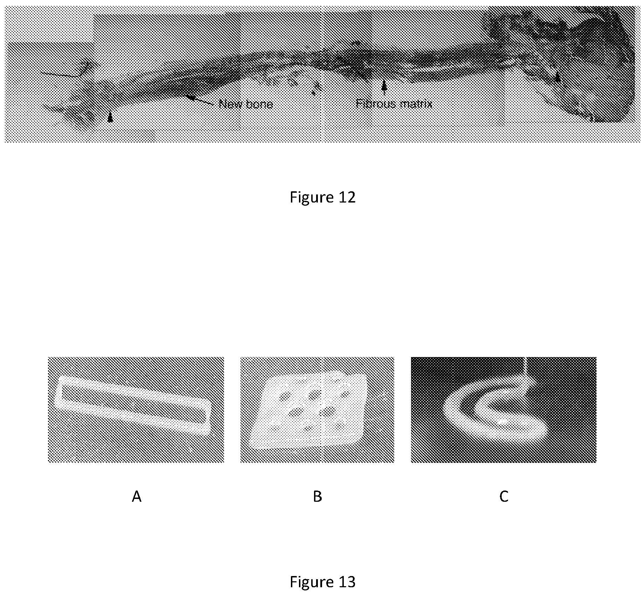

Healing was quantified using three-dimensional reconstruction of CT (Figure 12). Following the final CT scan in each experimental group, animals were sacrificed, and a sample of tissues was evaluated by conventional histology.

***Results***

One rat died soon after surgery and the other 5 were followed for 6 weeks and sacrificed. Bone was removed and subjected to X-Ray analysis for new bone formation and prepared for histology. One rat showed great regeneration for this interval (approximately 75%) while the other 4 showed between 10-25% regeneration. The tissue looked very healthy, and histology showed no signs of infiltration of macrophages, neutrophils or other inflammatory cells. Thus, it appears that abalone collagen can be cast as a native protein gel, which lacks inflammatory properties. The results indicate, abalone collagen could promote bone regeneration effectively at lower concentrations (3-10 mg/ml), which have proven efficacy with mammalian vertebrate collagens, and could be a useful carrier of stem cells and growth factors for bone tissue engineering.

Upon removal of the skin over the bone, it was clear that the abalone collagen is biocompatible. The tissues were healthy and non-inflamed, unlike rats we have observed in studies using gels of mouse laminin and reconstituted basement membrane, where the bone and associated soft tissues were inflamed and contained puss.

As depicted in the Figure 11, without treatment, the critical sized defect does not regenerate bone in the 6 weeks of the study. However, in the animals receiving abalone collagen gels, labeled A-E, there were a variety of degrees of bone regeneration observed from approximately 75% healing in rat A to 10-25 % healing in the other 4 rats. Collagen gels can be formed from MCol-B and such gels may promote bone regeneration.

### Example 13 MCol-B Patch for Rotator Cuff Repair

40 ml of 50mg/ml abalone collagen was frozen in liquid nitrogen and lyophilized to obtain a MCol-B patches. Once dried they were stored in a desiccator under vacuum at room temperature.

The lyophilised MCol-B fibril patch (MCol-FP) was fibrous and tough and could be a potential patch for Rotator Cuff Repair (RCR).

Rotator cuff patches are frequently referred to as ECM. An ECM is a complex structural entity that surrounds and supports mammalian cells and is the cell part of a tissue that is not part of any cell. The ECM is comprised of structural proteins (collagen), specialized proteins (fibrillin, fibronectin), and various proteoglycans. Biologic scaffolds composed of extracellular matrix (ECM) are commonly used for a variety of reconstructive surgical applications and are increasingly used in regenerative medicine strategies. However, DNA remnants have been found in many final products.

Rotator cuff ECM patches have been engineered to contain collagen as a scaffold from a variety of human and xenograft sources. Human sources include processed human dermal tissue, whereas animal sources include porcine small intestine submucosa (SIS), porcine dermis, and equine pericardium. Other graft sources have been described; however, the majority are derived from human dermis or porcine. Decellularized tissues should be verified to be substantially free of known toxic/deleterious materials such as residual cellular debris, lipids, nucleic acids, endotoxins, and known antigenic/immunogenic proteins.

Porcine small intestine submucosa was the earliest biological scaffold that was used in rotator cuff repair. Some of the scaffolds used for tendon and ligament reconstruction that are considered to be biological include Zimmer's Collagen Repair Patch (porcine dermal tissue), CuffPatch (porcine small intestine submucosa), Bio-Blanket (bovine dermis), and TissueMend® (fetal bovine dermis). However, these scaffolds are derived from mammalian sources and are cross-linked. Cross-linking is known to be able to provoke adverse effects within a biomaterial. Commercially available scaffolds for tendon repair are manufactured from animal or human connective tissues such as dermis, small intestine mucosa and pericardium. Antigenicity of collagen is related to the presence of non-collagenous proteins such as DNA and lipids. Polymerase Chain Reaction (PCR) analysis of the Restore graft demonstrated residual porcine DNA, and implantation in mice and rabbits confirmed host tissue response in the form of inflammatory changes and lymphocyte infiltration. Zimmer Collagen Repair Patch (porcine dermis) has mixed results, with studies reporting serious complications due to remanence of porcine DNA. Removal of non-collagenous components is critical to avoid foreign-body reaction or rejection by the host.

Smith et al (2017) assessed the mechanical properties of seven commercially available scaffolds for rotator cuff repair and compared those with the supraspinatus tendon. The testing showed that none of the available scaffolds have micro or macro mechanical properties similar to the supraspinatus tendon.

For a reinforcement patch to be efficacious in assisting in the tendon repair process, it needs to have a number of properties:

- Provide effective tension relief to the primary repair along the suture line for a sufficiently long duration to allow timely healing of the tendon/bone junction. This requires that reinforcement patches should have appropriate mechanical properties (load to failure, stiffness).

- Able to integrate with surrounding native tissues.

- Conducive to the healing process of the tendon and the re-establishment of the continuity of the tendon/bone junction.

- Biocompatible, sterile, and does not lead to inflammation of the surrounding tissues.

MCol-B is a highly pure non-cross-linked collagen and is therefore intrinsically highly resistant to cleavage by common proteases. Based on our biocompatibility data, MCol-B will exhibit the most desirable properties of a reinforcement patch.

### Example 14 3D Bioprinting

The bioink developed for this process has a collagen concentration of 12 mg/ml and a viscosity of 50 mPa.S when it is printed at temperature 4°C. Bioinks were prepared using both dialysed and non-dialysed abalone collagen.

The alignment of collagen fibrils in scaffolds is a critical parameter for control of architectural features. Without mimicking this alignment found in tissues, it is not possible to build a biomimetic tissue. The mechanical (i.e. functional) properties of most connective tissues are governed by the fibrous protein collagen and its 3D architecture. MCol-B was 3D bioprinted as a patch and provides suitable mechanical, physical, and biological properties. The bioprinted patch from MCol-B allows host cell integration and has natural porosity (providing an optimised environment for host-cell attachment, proliferation, and migration, while also assisting with gas and metabolite diffusion). These properties allow an MCol-B patch to quickly interact with host tissue and more rapidly induce new tissue formation. 3D bioprinting of abalone collagen fibril patch structurally mimics living tissues and the living scaffold with normal functioning in the long term.

Three-dimensional abalone collagen scaffolds of appropriate biological and biomechanical properties were bioprinted using the bioink. The bioprinter was a Cellink BIOX extrusion bioprinter. The bioprinter consists of an XY stage and a cartesian positioning system with three heads used to print different substrate types. All eight print heads use a pneumatic extruder to dispense the material onto the build platform. The volume of dispensed material was manipulated by controlling the pneumatic pressure, gauge and printing speed and temperature of the material. The parameters were adjusted using Cellink BIOX proprietary software.

The collagen stock was diluted to 12 mg/mL with MilliQ water and pH adjusted to 3.5. This solution was maintained at 4 °C until it was loaded into the cartridge for bioprinting. Patient specific Computer Aided Design (CAD) models can be created by converting computed tomography (CT) or magnetic resonance imaging (MRI) of clinical defects into (CAD) models.

Printability is the capability to form and maintain reproducible 3D constructs from bioink using the 3D bioprinting technique. Bioinks should maintain their shape once they leave the tip of the printing nozzle. Printability affects the structure of the printed scaffolds, and as a result, their mechanical and biological properties.

Owing to the poor printability of non-cross-linked hydrolysed collagen , printed constructs may collapse and fail to form a 3D structure. Highly cross-linked hydrolysed collagen can provide the shape fidelity after bioprinting but are not optimal from biological perspective due to impairment of cell migration and proliferation.

MCol-B bioink printability was assessed using the Cellink BIOX 3D bioprinter. MCol-B bioinks were prepared as described. The bioink was stored at 4 degree C until the bioink was loaded into a 3ml cartridge making sure there were no air bubbles. The loaded cartridge was maintained at 4 degree C by attaching the printer head temperature control. The temperature of the cartridge is closely related to the reaction kinetics. Printing was performed using a 25 gauge tapered nozzle and printed on the printer bed which was covered with parafilm as the printing surface. The printer bed was maintained at 24 degree C.

A number of different print models were printed successfully (Figure 13). Dialysed abalone collagen was used to print a line print (speed 15mm/s, pressure 10kPa and a meniscus (speed 15mm/s, pressure 11kPa) and non-dialysed abalone collagen was used to print a square with infills (11mm/s, pressure 12kPa) in a layer-by-layer (3 layers) fashion. The printed constructs were stable in their shape and size. The printed constructs were left to dry at room temperature and the dried constructs which retained its shape were peeled from the parafilm. The non-dialysed collagen is associated with the salt presence that provides greater rigidity to the 3D printed abalone collagen construct. While not wishing to be bound by theory, it is believed that non-covalent salt bridges that act as cross-linking points contribute to the strength of the association.

MCol-B bioink is a novel bioink for 3D biofabrication. MCol-B bioink allowed the fabrication of constructs with complicated geometry, retained the designed feature without moulding or chemical/photocrosslinking.

In describing and claiming the present invention, the following terminology will be used in accordance with the definitions set out below. It is also to be understood that the terminology used herein is for the purpose of describing particular embodiments of the invention only and is not intended to be limiting.

Unless defined otherwise, all technical and scientific terms used herein have the same meaning as commonly understood by one having ordinary skill in the art to which the invention pertains.

Unless the context clearly requires otherwise, throughout the description and the claims, the terms "comprise", "'comprising", and the like are to be construed in an inclusive sense as opposed to an exclusive or exhaustive sense; that is to say, in the sense of "including, but not limited to". For example, a composition, mixture, process or method that comprises a list of elements is not necessarily limited to only those elements but may include other elements not expressly listed or inherent to such composition, mixture, process or method.

The transitional phrase "consisting of" excludes any element, step, or ingredient not specified. If in the claim, such would close the claim to the inclusion of materials other than those recited except for impurities ordinarily associated therewith. When the phrase "consisting of" appears in a clause of the body of a claim, rather than immediately following the preamble, it limits only the element set forth in that clause; other elements are not excluded from the claim as a whole.

The transitional phrase "consisting essentially of" is used to define a composition, process or method that includes materials, steps, features, components, or elements, in addition to those literally disclosed, provided that these additional materials, steps, features, components, or elements do not materially affect the basic and novel characteristic(s) of the claimed invention. The term "consisting essentially of" occupies a middle ground between "comprising" and "consisting of".

Where applicants have defined an invention or a portion thereof with an open-ended term such as "comprising", it should be readily understood that (unless otherwise stated) the description should be interpreted to also describe such an invention using the terms "consisting essentially of" or "consisting of." In other words, with respect to the terms "comprising", "consisting of", and "consisting essentially of", where one of these three terms is used herein, the presently disclosed and claimed subject matter may include the use of either of the other two terms. Thus, in some embodiments not otherwise explicitly recited, any instance of "comprising" may be replaced by "consisting of" or, alternatively, by "consisting essentially of".

Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Also, the indefinite articles "a" and "an" preceding an element or component of the invention are intended to be non-restrictive regarding the number of instances (i.e., occurrences) of the element or component. Therefore "a" or "an" should be read to include one or at least one, and the singular word form of the element or component also includes the plural unless the number is obviously meant to be singular.

Other than in the operating examples, or where otherwise indicated, all numbers expressing quantities of ingredients or reaction conditions used herein are to be understood as modified in all instances by the term "about". The examples are not intended to limit the scope of the invention. In what follows, or where otherwise indicated, "%" will mean "weight %", "ratio" will mean "weight ratio" and "parts" will mean "weight parts".

The terms "predominantly" and "substantially" as used herein shall mean comprising more than 50% by weight, unless otherwise indicated.

As used herein, with reference to numbers in a range of numerals, the terms "about," "approximately" and "substantially" are understood to refer to the range of -10% to +10% of the referenced number, preferably -5% to +5% of the referenced number, more preferably -1 % to + 1 % of the referenced number, most preferably -0.1 % to +0.1 % of the referenced number. Moreover, with reference to numerical ranges, these terms should be construed as providing support for a claim directed to any number or subset of numbers in that range. For example, a disclosure of from 1 to 10 should be construed as supporting a range of from 1 to 8, from 3 to 7, from 1 to 9, from 3.6 to 4.6, from 3.5 to 9.9, from 8 to 10, and so forth.

As used herein, wt.% refers to the weight of a particular component relative to total weight of the referenced composition.

The term "and/or" used in the context of "X and/or Y" should be interpreted as "X," or "Y," or "X and Y." Similarly, "at least one of X or Y" should be interpreted as "X," or "Y," or "both X and Y."

Bailey A.J, Light N.D, and Atkins E.D.T. Chemical cross-linking restrictions on models for the molecular organization of the collagen fibre. Nature 1980; 288:408-410.

Cho JK, Jin YG, Rha SJ, Kim SJ, Hwang JH. Biochemical characteristics of four marine fish skins in Korea. Food Chem 2014;159: 200-207.

Eyre D.R. and Oguchi H. The hydroxypyridinium cross-link of skeletal collagen. Biochem. Biophys. Res. Commun. 1980; 92:403-410.

Fauzi M.B, Lokanathan Y, Aminuddin B.S, Ruszymah B.H.I, Chowdhury S.R. Ovine tendon collagen: Extraction, characterisation and fabrication of thin films for tissue engineering applications. Mater. Sci. Eng. C 2016;68:163-171.

Gopal Shankar K, Udhaya Kumar S, Sowndarya S, Suresh Babu P, Rose C. Isolation, characterization, and in vitro evaluation of bovine rumen submucosa films of collagen or chitosan-treated collagen. J Biomater Appl. 2015;30(6):780- 92.

Habermehl J, Skopinska J, Boccafoschi F, et al. Preparation of ready-to-use, stockable and reconstituted collagen. Macromol Biosci. 2005;5(9):821-8.

Knott L, and Bailey A.J. (1998) Collagen cross-links in mineralising tissues: a review of their chemistry, function and clinical relevance. Bone 1998; 22:181-187.

Leikina E, Mertts M.V, N. Kuznetsova, and S. Leikin Type I collagen is thermally unstable at body temperature. Proceedings of the National Academy of Sciences 2002; 99:1314-1318.

Li Z, Wang B, Chi C, Gong Y, Luo H, Ding G. Influence of average molecular weight on antioxidant and functional properties of cartilage collagen hydrolysates from Sphyrna Lewini, Dasyatis Akjei and Raja Porosa. Food Res. Int. 2013; 51:283-293.

Norma J. Greenfield Using circular dichroism spectra to estimate protein secondary structure. Nat Protoc. 2006; 1(6): 2876-2890.

Patel NR and Gohil PP. A review on biomaterials: scope, applications & human anatomy significance. International Journal of Emerging Technology and Advanced Engineering 2012; 2(4): 91-101.

Ramadass S.K, Perumal S, Gopinath A, Nisal A, Subramanian S, Madhan B. Sol-gel assisted fabrication of collagen hydrolysate composite scaffold: A novel therapeutic alternative to the traditional collagen scaffold. Acs Appl. Mater. Interfaces 2014; 6:15015-15025.

Rosenblo J, Harsch M, Jimenez, S. Hydroxyproline content determines denaturation temperature of chick tendon collagen. Arch. Biochem. Biophys. 1973; 158:478-484.

Rubin AL, Stenzel KH, Miyata T, White MJ, Dunn M. Collagen as a vehicle for drug delivery. Preliminary report. J Clin Pharmacol. 1973;13(8):309-12.

Sun Pan B, En Chen H.O.A, Sung W.C. Molecular and thermal characteristics of acid-soluble collagen from orbicular batfish: Effects of deep-sea water culturing. Int. J. Food Prop. 2018; 21:1080-1090.

Smith-Mungo L.I, and Kagan H.M. Lysyl oxidase: properties, regulation and multiple functions in biology. Matrix Biol 1998; 16:387-398.

Smith RDJ, Zargar N, Brown CP, et al. Characterizing the macro and micro mechanical properties of scaffolds for rotator cuff repair. J Shoulder Elbow Surg 2017; 26:2038-2046.

Tathe A, Ghodke M and Nikalje AP. A brief review: Biomaterials and their application. International Journal of Pharmacy and Pharmaceutical Sciences, 2010;2(4):19-23.

Yamauchi M, London R.E, Guemat C, Hashimoto F, and Mechanic G.L. Structure and function of a stable histidine- based tri-functional cross-link in skin collagen. J. Biol. Chem. 1987; 262:11428-11434.

Zhang Y, Zhang Y, Liu X, Huang L, Chen, Z, Cheng J. Influence of hydrolysis behaviour and microfluidisation on the functionality and structural properties of collagen hydrolysates. Food Chem. 2017; 227:211-218.

**Claims**

1. A method of preparing a manufactured article, comprising the steps of:

   a) providing a plurality of isolated and purified type I collagen fibrils derived from abalone;
   b) forming the isolated and purified type I collagen fibrils into a manufactured article which contains ions or salts, preferably sodium chloride, within an intrafibrillar compartment.

2. A method as claimed in claim 1, wherein the manufactured article is formed by:

   a) dissolving the isolated and purified type I collagen fibrils to produce a bioink with a predetermined viscosity;
   b) introducing the collagen bioink to a 3D bioprinter;
   c) printing the manufactured article in the 3D bioprinter while maintaining a relatively constant temperature to maintain the predetermined viscosity.

3. A method as claimed in claim 2, wherein:

   • the temperature that is maintained is greater than 0°C and less than or equal to 15°C, preferably 4°C;
   • the viscosity of the collagen solution is from 10mPa-S to 60mPa-S; and/or
   • the concentration of the collagen solution is from 3mg/ml to 20mg/ml.

4. A method as claimed in claim 1, wherein the manufactured article is formed by:

   a) dissolving the isolated and purified type I collagen fibrils to produce a collagen solution at room temperature or below;
   b) shaping the collagen solution; and
   c) inducing polymerisation of collagen molecules to form a hydrated gel;

   wherein steps (a) and (b) are carried out at a temperature below that at which the collagen molecules polymerise, preferably below room temperature and more preferably at 4°C, and polymerisation is induced by way of an increase in temperature, preferably to a temperature of greater than 4°C and less than or equal to 37°C.

5. A method as claimed in claim 4, wherein the pH of the collagen solution produced in step (a) is 3.5.

6. A method as claimed in either one of claims 4 or 5, wherein the pH of the collagen solution is adjusted towards physiological pH prior to shaping the article by the addition of a base, preferably to 7.4 ± 10%.

7. A method as claimed in any one of claims 4 to 6, wherein the collagen solution has a concentration of greater than 35mg/ml.

8. A method as claimed in any one of claims 4 to 7, wherein the collagen solution is cast as a sheet or moulded.

9. A method as claimed in any one of claims 4-to 8, wherein the manufactured article is for implantation into an animal such as a scaffold.

10. A bioink for a 3D bioprinter comprising isolated and purified type I collagen fibrils derived from abalone and a solvent, wherein the bioink has a predetermined concentration of abalone collagen fibrils and a predetermined viscosity that is low enough that the bioink can be bioprinted at a temperature below the denaturation temperature of abalone collagen and wherein a printed article contains ions or salts within an intrafibrillar compartment.

11. A bioink as claimed in claim 10, wherein the viscosity of the collagen bioink is from 10mPa-S to 60mPa-S.

12. A bioink as claimed in either one of claims 10 or 11, wherein the solvent is an aqueous solution of a weak acid and, preferably, the pH of the solution is about 3.5.

13. A cartridge containing a bioink as claimed in any one of claims 10 to 12, wherein the cartridge is adapted to fit into a 3D bioprinter to provide bioink for bioprinting.

14. A manufactured article such as a scaffold for tissue engineering, comprising a plurality of isolated and purified type I collagen fibrils derived from abalone in which there is association of collagen molecules from adjacent collagen fibrils and which contains ions or salts, preferably sodium chloride, within an intrafibrillar compartment.

15. A method of isolating type I collagen fibrils from abalone, comprising the steps of:

   a) preparing a collagen-containing portion of abalone for extraction;
   b) contacting the collagen-containing portion with a weak acid solution to provide an acidic type I collagen fibril solution;
   c) separation insoluble material from the acidic type I collagen fibril solution;
   d) adjusting the pH of the acidic type I collagen fibril solution to a pH just below the isoelectric point of abalone collagen, preferably to 4.0 $\pm$ 10%;
   e) precipitating native type I collagen fibrils by addition of a salt, preferably sodium chloride;

   wherein the precipitated native type I collagen fibrils are not dialysed against water.


**Patentansprüche**

1. Verfahren zur Herstellung eines hergestellten Artikels, umfassend die Schritte:

   a) Bereitstellen einer Vielzahl von isolierten und gereinigten Typ-I-Kollagenfibrillen, die von Abalone abgeleitet sind;
   b) Formen der isolierten und gereinigten Typ-I-Kollagenfibrillen zu einem hergestellten Artikel, der Ionen oder Salze, vorzugsweise Natriumchlorid, in einem intrafibrillären Kompartiment enthält.

2. Verfahren nach Anspruch 1, wobei der hergestellte Artikel geformt wird durch:

   a) Auflösen der isolierten und gereinigten Typ-I-Kollagenfibrillen, um eine Biotinte mit einer vorbestimmten Viskosität herzustellen;
   b) Einführen der Kollagenbiotinte in einen 3D-Biodrucker;
   c) Drucken des hergestellten Artikels in dem 3D-Biodrucker, während eine relativ konstante Temperatur aufrechterhalten wird, um die vorbestimmte Viskosität aufrechtzuerhalten.

3. Verfahren nach Anspruch 2, wobei:

   • die Temperatur, die aufrechterhalten wird, größer als 0 °C und kleiner als oder gleich 15 °C, vorzugsweise 4 °C, ist;
   • die Viskosität der Kollagenlösung von 10 mPa-S bis 60 mPa-S beträgt; und/oder
   • die Konzentration der Kollagenlösung von 3 mg/ml bis 20 mg/ml beträgt.

4. Verfahren nach Anspruch 1, wobei der hergestellte Artikel geformt wird durch:

   a) Auflösen der isolierten und gereinigten Typ-I-Kollagenfibrillen, um eine Kollagenlösung bei Raumtemperatur oder darunter herzustellen;
b) Formen der Kollagenlösung; und
c) Induzieren der Polymerisation von Kollagenmolekülen, um ein hydratisiertes Gel zu bilden;

wobei die Schritte (a) und (b) bei einer Temperatur unterhalb derjenigen, bei der die Kollagenmoleküle polymerisieren, vorzugsweise unterhalb Raumtemperatur und stärker bevorzugt bei 4 °C, durchgeführt werden und die Polymerisation durch einen Temperaturanstieg, vorzugsweise auf eine Temperatur von größer als 4 °C und kleiner als oder gleich 37 °C, induziert wird.

5. Verfahren nach Anspruch 4, wobei der pH-Wert der in Schritt (a) hergestellten Kollagenlösung 3,5 beträgt.

6. Verfahren nach einem der Ansprüche 4 oder 5, wobei der pH-Wert der Kollagenlösung vor dem Formen des Artikels durch die Zugabe einer Base, vorzugsweise auf 7,4 ± 10 %, in Richtung des physiologischen pH-Werts eingestellt wird.

7. Verfahren nach einem der Ansprüche 4 bis 6, wobei die Kollagenlösung eine Konzentration von mehr als 35 mg/ml aufweist.

8. Verfahren nach einem der Ansprüche 4 bis 7, wobei die Kollagenlösung als Folie gegossen oder geformt wird.

9. Verfahren nach einem der Ansprüche 4 bis 8, wobei der hergestellte Artikel zur Implantation in ein Tier, wie zum Beispiel ein Gerüst, ist.

10. Biotinte für einen 3D-Biodrucker, umfassend isolierte und gereinigte Typ-I-Kollagenfibrillen, die von Abalone abgeleitet sind, und ein Lösungsmittel, wobei die Biotinte eine vorbestimmte Konzentration von Abalone-Kollagenfibrillen und eine vorbestimmte Viskosität aufweist, die niedrig genug ist, dass die Biotinte bei einer Temperatur unter der Denaturierungstemperatur von Abalone-Kollagen biogedruckt werden kann, und wobei ein gedruckter Artikel Ionen oder Salze in einem intrafibrillären Kompartiment enthält.

11. Biotinte nach Anspruch 10, wobei die Viskosität der Kollagenbiotinte von 10 mPa-S bis 60 mPa-S beträgt.

12. Biotinte nach einem der Ansprüche 10 oder 11, wobei das Lösungsmittel eine wässrige Lösung einer schwachen Säure ist und der pH-Wert der Lösung vorzugsweise etwa 3,5 beträgt.

13. Kartusche, die eine Biotinte nach einem der Ansprüche 10 bis 12 enthält, wobei die Kartusche dafür ausgelegt ist, in einen 3D-Biodrucker zu passen, um Biotinte für das Biodrucken bereitzustellen.

14. Hergestellter Artikel, wie etwa ein Gerüst für das Tissue Engineering, umfassend eine Vielzahl von isolierten und gereinigten Typ-I-Kollagenfibrillen, die von Abalone abgeleitet sind, wobei es eine Assoziation von Kollagenmolekülen von benachbarten Kollagenfibrillen gibt und die Ionen oder Salze, vorzugsweise Natriumchlorid, in einem intrafibrillären Kompartiment enthält.

15. Verfahren zum Isolieren von Typ-I-Kollagenfibrillen aus Abalone, umfassend die Schritte:

a) Herstellen eines kollagenhaltigen Abschnitts von Abalone zur Extraktion;
b) Inkontaktbringen des kollagenhaltigen Abschnitts mit einer schwachen Säurelösung, um eine saure Typ-I-Kollagenfibrillenlösung bereitzustellen;
c) Trennen von unlöslichem Material von der sauren Typ-I-Kollagenfibrillenlösung;
d) Einstellen des pH-Werts der sauren Typ-I-Kollagenfibrillenlösung auf einen pH-Wert knapp unterhalb des isoelektrischen Punkts von Abalone-Kollagen, vorzugsweise auf etwa 4,0 ± 10 %;
e) Ausfällen von nativen Typ-I-Kollagenfibrillen durch Zugabe eines Salzes, vorzugsweise Natriumchlorid;

wobei die ausgefällten nativen Typ-I-Kollagenfibrillen nicht gegen Wasser dialysiert werden.

**Revendications**

1. Procédé de préparation d'un article manufacturé, comprenant les étapes de :

a) fourniture d'une pluralité de fibrilles de collagène de type I purifiées et isolées dérivées d'ormeau ;
b) formation des fibrilles de collagène de type I purifiées et isolées en un article manufacturé qui contient des ions ou des sels, de préférence du chlorure de sodium, à l'intérieur d'un compartiment intrafibrillaire.

2. Procédé selon la revendication 1, dans lequel l'article manufacturé est formé par :

a) dissolution des fibrilles de collagène de type I purifiées et isolées pour produire une bio-encre ayant une viscosité prédéfinie ;
b) introduction de la bio-encre de collagène dans une bio-imprimante 3D ;
c) impression de l'article manufacturé dans la bio-imprimante 3D tout en maintenant une température relativement constante afin de maintenir la viscosité prédéfinie.

3. Procédé selon la revendication 2, dans lequel :

- la température qui est maintenue est supérieure à 0°C et inférieure ou égale à 15°C, de préférence 4°C ;
- la viscosité de la solution de collagène est de 10 mPa-s à 60 mPa-s ; et/ou
- la concentration de la solution de collagène est de 3 mg/ml à 20 mg/ml.

4. Procédé selon la revendication 1, dans lequel l'article manufacturé est formé par :

a) dissolution des fibrilles de collagène de type I purifiées et isolées pour produire une solution de collagène à température ambiante ou en dessous ;
b) mise en forme de la solution de collagène ; et
c) induction de la polymérisation des molécules de collagène pour former un gel hydraté ;

dans lequel les étapes (a) et (b) sont réalisées à une température inférieure à celle à laquelle les molécules de collagène polymérisent, de préférence inférieure à la température ambiante et de manière mieux préférée à 4°C, et la polymérisation est induite au moyen d'une hausse de la température, de préférence à une température supérieure à 4°C et inférieure ou égale à 37°C.

5. Procédé selon la revendication 4, dans lequel le pH de la solution de collagène produite à l'étape (a) est de 3,5.

6. Procédé selon l'une quelconque des revendications 4 ou 5, dans lequel le pH de la solution de collagène est ajusté vers le pH physiologique avant la mise en forme de l'article par l'addition d'une base, de préférence à 7,4 ± 10 %.

7. Procédé selon l'une quelconque des revendications 4 à 6, dans lequel la solution de collagène a une concentration supérieure à 35 mg/ml.

8. Procédé selon l'une quelconque des revendications 4 à 7, dans lequel la solution de collagène est coulée en tant que feuille ou moulée.

9. Procédé selon l'une quelconque des revendications 4 à 8, dans lequel l'article manufacturé est destiné à l'implantation chez un animal en tant qu'échaffaudage.

10. Bio-encre pour une bio-imprimante 3D comprenant des fibrilles de collagène de type I purifiées et isolées dérivées d'ormeau et un solvant, où la bio-encre a une concentration prédéfinie en fibrilles de collagène d'ormeau et une viscosité prédéfinie qui est suffisamment faible pour que la bio-encre puisse être bio-imprimée à une température inférieure à la température de dénaturation du collagène d'ormeau et où un article imprimé contient des ions ou des sels à l'intérieur d'un compartiment intrafibrillaire.

11. Bio-encre selon la revendication 10, dans laquelle la viscosité de la bio-encre de collagène va de 10 mPa-s à 60 mPa-s.

12. Bio-encre selon l'une quelconque des revendications 10 ou 11, dans laquelle le solvant est une solution aqueuse d'acide faible et, de préférence, le pH de la solution est d'environ 3,5.

13. Cartouche contenant une bio-encre selon l'une quelconque des revendications 10 à 12, où la cartouche est adaptée à s'emboîter dans une bio-imprimante 3D pour fournir une bio-encre pour la bio-impression.

**14.** Article manufacturé tel qu'un échaffaudage pour le génie tissulaire, comprenant une pluralité de fibrilles de collagène de type I purifiées et isolées dérivées d'ormeau dans lequel il y a une association de molécules de collagène provenant de fibrilles de collagène adjacentes et qui **contient** des ions ou des sels, de préférence du chlorure de sodium, à l'intérieur d'un compartiment intrafibrillaire.

**15.** Procédé d'isolation de fibrilles de collagène de type I à partir d'ormeau, comprenant les étapes de :

a) préparation d'une portion d'ormeau contenant du collagène pour l'extraction ;

b) mise en contact de la portion contenant du collagène avec une solution d'acide faible afin de fournir une solution acide de fibrilles de collagène de type I ;

c) séparation de la matière insoluble et de la solution acide de fibrilles de collagène de type I ;

d) ajustement du pH de la solution acide de fibrilles de collagène de type I à un pH juste en dessous du point isoélectrique du collagène d'ormeau, de préférence à 4,0 ± 10 % ;

e) précipitation des fibrilles de collagène de type I natif par addition d'un sel, de préférence du chlorure de sodium ;

dans lequel les fibrilles de collagène de type I natif précipitées ne sont pas dialysées contre de l'eau.

Figure 1

Figure 2

Figure 3

Figure 4

Figure 5

A: NIH-3T3

B: BHK

Figure 6

A: hASC

B: BHK

Figure 7

MCol-B no serum      Bovine collagen no serum

MCol-B with serum      Bovine collagen with serum

Figure 8

A. BKH

Figure 9

B. NIH-3T3

Figure 9  (continued)

C. hASC

Figure 9  (continued)

Figure 10

Untreated

MCol-B Gel1(5mg/ml)

Figure 11

Figure 12

A                                B                                C

Figure 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040167318 A1

- WO 2002102831 A

**Non-patent literature cited in the description**

- **BAILEY A.J** ; **LIGHT N.D** ; **ATKINS E.D.T**. Chemical cross-linking restrictions on models for the molecular organization of the collagen fibre. *Nature*, 1980, vol. 288, 408-410
- **CHO JK** ; **JIN YG** ; **RHA SJ** ; **KIM SJ** ; **HWANG JH**. Biochemical characteristics of four marine fish skins in Korea. *Food Chem*, 2014, vol. 159, 200-207
- **EYRE D.R** ; **OGUCHI H**. The hydroxypyridinium cross-link of skeletal collagen. *Biochem. Biophys. Res. Commun.*, 1980, vol. 92, 403-410
- **FAUZI M.B** ; **LOKANATHAN Y** ; **AMINUDDIN B.S** ; **RUSZYMAH B.H.I** ; **CHOWDHURY S.R**. Ovine tendon collagen: Extraction, characterisation and fabrication of thin films for tissue engineering applications. *Mater. Sci. Eng. C*, 2016, vol. 68, 163-171
- **GOPAL SHANKAR K** ; **UDHAYA KUMAR S** ; **SOWNDARYA S** ; **SURESH BABU P** ; **ROSE C. ISOLATION**. characterization, and in vitro evaluation of bovine rumen submucosa films of collagen or chitosan-treated collagen. *J Biomater Appl.*, 2015, vol. 30 (6), 780-92
- **HABERMEHL J** ; **SKOPINSKA J** ; **BOCCAFOSCHI F et al.** Preparation of ready-to-use, stockable and reconstituted collagen. *Macromol Biosci.*, 2005, vol. 5 (9), 821-8
- **KNOTT L** ; **BAILEY A.J** ; **1998**. Collagen cross-links in mineralising tissues: a review of their chemistry, function and clinical relevance. *Bone*, 1998, vol. 22, 181-187
- **LEIKINA E** ; **MERTTS M.V** ; **N. KUZNETSOVA** ; **S. LEIKIN**. Type I collagen is thermally unstable at body temperature.. *Proceedings of the National Academy of Sciences*, 2002, vol. 99, 1314-1318
- **LI Z** ; **WANG B** ; **CHI C** ; **GONG Y** ; **LUO H** ; **DING G**. Influence of average molecular weight on antioxidant and functional properties of cartilage collagen hydrolysates from Sphyrna Lewini, Dasyatis Akjei and Raja Porosa. *Food Res. Int.*, 2013, vol. 51, 283-293
- **NORMA J.** Greenfield Using circular dichroism spectra to estimate protein secondary structure. *Nat Protoc.*, 2006, vol. 1 (6), 2876-2890

- **PATEL NR** ; **GOHIL PP**. A review on biomaterials: scope, applications & human anatomy significance. *International Journal of Emerging Technology and Advanced Engineering*, 2012, vol. 2 (4), 91-101
- **RAMADASS S.K** ; **PERUMAL S** ; **GOPINATH A** ; **NISAL A** ; **SUBRAMANIAN S** ; **MADHAN B**. Sol-gel assisted fabrication of collagen hydrolysate composite scaffold: A novel therapeutic alternative to the traditional collagen scaffold. *Acs Appl. Mater. Interfaces*, 2014, vol. 6, 15015-15025
- **ROSENBLO J** ; **HARSCH M** ; **JIMENEZ, S**. Hydroxyproline content determines denaturation temperature of chick tendon collagen. *Arch. Biochem. Biophys.*, 1973, vol. 158, 478-484
- **RUBIN AL** ; **STENZEL KH** ; **MIYATA T** ; **WHITE MJ** ; **DUNN M**. Collagen as a vehicle for drug delivery. *Preliminary report. J Clin Pharmacol.*, 1973, vol. 13 (8), 309-12
- **SUN PAN B** ; **EN CHEN H.O.A** ; **SUNG W.C**. Molecular and thermal characteristics of acid-soluble collagen from orbicular batfish: Effects of deep-sea water culturing. *Int. J. Food Prop*, 2018, vol. 21, 1080-1090
- **SMITH-MUNGO L.I** ; **KAGAN H.M**. Lysyl oxidase: properties, regulation and multiple functions in biology. *Matrix Biol*, 1998, vol. 16, 387-398
- **SMITH RDJ** ; **ZARGAR N** ; **BROWN CP et al.** Characterizing the macro and micro mechanical properties of scaffolds for rotator cuff repair. *J Shoulder Elbow Surg*, 2017, vol. 26, 2038-2046
- **TATHE A** ; **GHODKE M** ; **NIKALJE AP**. A brief review: Biomaterials and their application. *International Journal of Pharmacy and Pharmaceutical Sciences*, 2010, vol. 2 (4), 19-23
- **YAMAUCHI M** ; **LONDON R.E** ; **GUEMAT C** ; **HASHIMOTO F** ; **MECHANIC G.L**. Structure and function of a stable histidine- based tri-functional cross-link in skin collagen. *J. Biol. Chem.*, 1987, vol. 262, 11428-11434

- **ZHANG Y** ; **ZHANG Y** ; **LIU X** ; **HUANG L** ; **CHEN, Z** ; **CHENG J**. Influence of hydrolysis behaviour and microfluidisation on the functionality and structural properties of collagen hydrolysates. *Food Chem.*, 2017, vol. 227, 211-218